# EUROPÄISCHE PATENTSCHRIFT

(11) **EP 0 617 891 B1**
(45) Veröffentlichungstag und Bekanntmachung des Hinweises auf die Patenterteilung: **28.05.1997**
(21) Anmeldenummer: 94103358.1
(22) Anmeldetag: 05.03.1994
(51) Int. Cl.: A01N 43/40, C07D 213/74

(54) **Verfahren zur Schädlingsbekämpfung und 2-Anilinopyridine**
Process for combatting agricultural pests and 2-anilin-pyridines
Procédé pour le combat des moisissures agricoles et 2-aniline-pyridines.

(30) Priorität: 17.03.1993 DE 4308395
(43) Veröffentlichungstag der Anmeldung: 05.10.1994
(73) Patentinhaber: BASF Aktiengesellschaft, 67063 Ludwigshafen (DE)
(72) Erfinder: Wagner, Oliver, Dr., D-66450 Bexbach (DE); Eicken, Karl, Dr., D-67157 Wachenheim (DE); Ammermann, Eberhard, Dr., D-64646 Heppenheim (DE); Lorenz, Gisela, Dr., D-67434 Neustadt (DE)

(56) Entgegenhaltungen:
- EP-A- 0 384 311
- EP-A- 0 401 168
- DE-A- 3 731 626
- CHEMICAL ABSTRACTS, vol. 69, no. 23, 2. Dezember 1968, Columbus, Ohio, US; abstract no. 96598z, R. FUSCO, P. DALLA CROCE & A. SALVI. 'Reactions of alpha-arylazo-alpha-chloroacetic acid esters with cyclic teriary bases.' Seite 9042 ;

## Beschreibung

Die vorliegende Erfindung betrifft ein Verfahren zur Bekämpfung von Schädlingen, welches dadurch gekennzeichnet ist, daß man die Schädlinge oder die von Befall mit Schädlingen bedrohten Pflanzen oder ihre Umgebung oder ihre Saatgüter oder vor Schädlingsbefall zu schützenden Materialien mit einer wirksamen Menge eines Wirkstoffs behandelt, der ein 2-Anilinopyridin der Formel I ist, 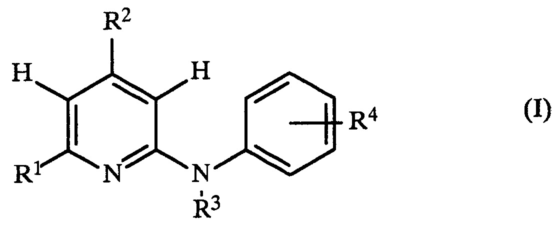 in der die Substituenten die folgenden Bedeutungen haben:
- R¹: C₁-C₆-Alkyl, C₂-C₄-Alkenyl, C₂-C₄-Alkinyl, C₁-C₆-Halogenalkyl, C₁-C₆-Alkoxy-C₁-C₆-alkyl, C₁-C₆-Alkylthio-C₁-C₆-alkyl oder C₃-C₆-Cycloalkyl;
durch C₁-C₂-Alkyl oder Halogen bis zu dreifach gleich oder verschieden substituiertes C₃-C₆-Cycloalkyl, C₁-C₆-Alkoxy oder C₁-C₆-Halogenalkoxy;
durch Hydroxy substituiertes C₁-C₂-Alkyl, C₂-C₄-Alkenyloxy oder C₂-C₄-Alkinyloxy;
Halogen, CN, SCN, Formyl, CH=NOR⁵, CH=NR⁶, CH₂NHR⁶
R⁵ Wasserstoff, C₁-C₈-Alkyl;
C₁-C₄-Alkyl, das durch Halogen, Hydroxy, Cyano, C₁-C₄-Alkoxy, COOC₁-C₃-Alkyl, oder durch Phenyl substituiert ist, wobei das Phenyl durch Halogen, C₁-C₃-Alkyl, C₁-C₃-Alkoxy, oder Nitro substituiert sein kann;
C₃-C₆-Alkenyl, durch Halogen substituiertes C₃-C₆-Alkenyl, C₃-C₆-Alkinyl, durch Halogen substituiertes C₃-C₆-Alkinyl,
Phenyl oder 1 bis 3-fach durch Halogen, C₁-C₃-Alkyl, C₁-C₃-Alkoxy oder Nitro substituiertes Phenyl oder
einen Acylrest COR⁷, worin
R⁷ C₁-C₆-Alkyl oder durch Halogen oder C₁-C₃-Alkoxy substituiertes C₁-C₆-Alkyl, C₃-C₆-Alkenyl, oder durch Halogen substituiertes C₃-C₆-Alkenyl bedeutet;
R⁶ Wasserstoff, C₁-C₆-Alkyl,
C₁-C₄-Alkyl, das durch Halogen, Hydroxy, Cyano, C₁-C₄-Alkoxy, C₁-C₄-Alkylamino, C₃-C₆-Cycloalkyl, oder durch Phenyl substituiert ist, wobei das Phenyl durch Halogen, C₁-C₃-Alkyl, C₁-C₃-Alkoxy, oder Nitro substituiert sein kann;
C₃-C₆-Cycloalkyl, oder durch Methyl substituiertes C₃-C₆-Cycloalkyl; C₃-C₆-Alkenyl oder durch Halogen substituiertes C₃-C₆-Alkenyl; C₃-C₆-Alkinyl, oder durch Halogen substituiertes C₃-C₆-Alkinyl;
Phenyl oder durch Halogen, C₁-C₃-Alkyl, C₁-C₃-Alkoxy, C₁-C₂-Halogenalkyl, C₁-C₂-Halogenalkoxy, oder Nitro substituiertes Phenyl;
- R²: C₁-C₆-Alkyl, C₂-C₄-Alkenyl, C₂-C₄-Alkinyl, C₁-C₆-Halogenalkyl, C₃-C₆-Cycloalkyl, durch C₁-C₂-Alkyl oder Halogen bis zu dreifach gleich oder verschieden substituiertes C₃-C₆-Cycloalkyl,
- R³: Wasserstoff, CN, S(O)ₙR⁸, wobei n = 0, 1, 2 bedeutet, oder COR⁹
- R⁸: C₁-C₃-Alkyl, Phenyl, gegebenenfalls 1- bis 3-fach durch Halogen, C₁-C₂-Alkyl, C₁-C₂-Halogenalkyl, Methoxy, Nitro substituiert,
- R⁹: Wasserstoff, C₁-C₆-Alkyl, C₁-C₆-Halogenalkyl, C₃-C₆-Cycloalkyl, Phenyl oder Benzyl, oder 1 bis 3-fach mit Halogen, C₁-C₃-Alkyl, C₁-C₃-Halogenalkyl, C₁-C₃-Alkoxy, Nitro oder Cyano substituiertes Phenyl oder durch diese Reste substituiertes Benzyl,
- R⁴: Wasserstoff, Halogen, C₁-C₃-Alkyl, C₁-C₂-Halogenalkyl, C₁-C₃-Alkoxy oder C₁-C₃-Halogenalkoxy, Cyano.

Außerdem betrifft die Erfindung die Verwendung der Verbindungen I zur Herstellung von Mitteln gegen Schädlinge und derartige Mittel zur Bekämpfung von Pilzen.

Die einzelnen Reste bedeuten beispielsweise:

Halogen steht für: Fluor, Chlor, Brom, Jod.

Unter Alkyl selbst oder als Bestandteil eines anderen Substituenten, wie Halogenalkyl, Alkoxy, Halogenalkoxy sind je nach Anzahl der benannten Kohlenstoffatome beispielsweise zu verstehen: Methyl, Ethyl, Propyl, 1-Methylethyl, Butyl, 1-Methylpropyl, 2-Methylpropoyl, 1,1-Dimethylethyl, n-Pentyl, 1-Methylbutyl, 2-Methylbutyl, 3-Methylbutyl, 1,2-Dimethylpropyl, 1,1-Dimethylpropyl, 2,2-Dimethylpropyl, 1-Ethylpropyl, n-Hexyl, 1-Methylpentyl, 2-Methylpentyl, 3-Methylpentyl, 4-Methylpentyl, 1,2-Dimethylbutyl, 1,3-Dimethylbutyl, 2,3-Dimethylbutyl, 1,1-Dimethylbutyl, 2,2-Dimethylbutyl, 3,3-Dimethyl-butyl, 1,1,2-Trimethylpropyl, 1,2,2-Trimethylpropyl, 1-Ethylbutyl, 2-Ethylbutyl, 1-Ethyl-2-methylpropyl,besonders bevorzugt Methyl,wobei das Alkyl ein bis drei Halogenatome tragen kann wie z.B. CHCl₂CH₂F, CCl₃, CH₂Cl, CHF₂, CH₂CH₂Br, C₂Cl₅, CH₂Br, CHBrCl vorzugsweise CF₃.

Cycloalkyl steht für Cyclopropyl, Cyclobutyl, Cyclopentyl, Cyclohexyl, besonders bevorzugt Cyclopropyl, wobei das Cycloalkyl gegebenenfalls durch 1-3 C₁-C₂-Alkylreste oder Halogen substituiert sein kann.

Alkenyl bedeutet z.B. Propenyl-(1), Allyl, Butenyl-(1), Butenyl-(2), Butenyl-(3), 1-Methyl-2-propenyl, 2-Methyl-2-propenyl,
Alkinyl bedeutet z.B. Propinyl-(1),Propinyl-(2), Butinyl-(1), Butinyl-(2), vorzugsweise Propinyl-(1).

Als besonders bevorzugte Einzelverbindungen für die Anwendung sind die folgenden zu nennen:
2-Anilino-4,6-dimethyl-pyridin,
2-(3-Fluoranilino)-4,6-dimethyl-pyridin,
2-Anilin-6-cyclopropyl-4-methyl-pyridin,
2-(3-Fluoranilino)-6-cyclopropyl-4-methyl-pyridin.

Ferner betrifft die Erfindung die Anilinopyridine der Formel I, ausgenommen diejenigen Verbindungen, in denen
a) gleichzeitig R¹ = C₁-C₄-Alkyl, C₁-C₄-Alkoxy, C₁-C₄-Halogenalkyl, C₁-C₄-Halogenalkoxy, C₃-C₆-Cycloalkyl, Halogen oder Cyano, R² = C₁-C₄-Alkyl, C₁-C₄-Halogenalkyl oder C₃-C₆-Cycloalkyl und R³ = Wasserstoff, und
b) R¹ = Methyl, R² = Methyl, R³ = Cyano und R⁴ = Wasserstoff.

Es ist bekannt, daß 2-Anilino-3-cyano-pyridine (DE-A 3 731 626) fungizide Wirkung besitzen. Ihre Wirkung ist jedoch nicht befriedigend. Ferner sind N-Phenyl-N-pyridin-2-ylharnstoffe mit herbizider und pflanzenwuchsregulierender Wirkung bekannt (EP-A 401 168). Für sie ist jedoch keine fungizide Wirkung beschrieben.

Es wurde nun überraschend gefunden, daß 2-Anilinopyridine der Formel I gemäß Anspruch 1 eine starke Wirkung gegen Schädlinge, insbesondere Pilze, insbesondere phytopathogene Pilzen, insbesondere Botrytis cinerea besitzen.

Die Herstellung der Verbindungen der allgemeinen Formel I erfolgt beispielsweise wie in den folgenden Schemata beschrieben:

Die Verbindungen der Formel I erhält man beispielsweise, indem man die Pyridin-N-oxide 1a mit den jeweiligen Anilinderivaten Ib in Gegenwart von Alkyl- oder Arylsulfonylchloriden, bevorzugt Tosylchlorid, in Gegenwart eines basischen Katalysators wie z.B. Alkalimetallhydroxide, bevorzugt Natriumhydroxid oder Kaliumhydroxid, zur Reaktion bringt. Die sauer katalysierte Hydrolyse der N-Tosylanilinopyridine Ic liefert die Anilinopyridine I (vgl. z.B.: Yu. V. Kurbatov, M.A. Solekhova, Khim. Getero. Soedin., 7 (1986) 936).

Die Vorprodukte Ia sind teilweise bekannt. Ihre Herstellung erfolgt nach in der Literatur beschriebenen Verfahren (siehe z.B. R. A. Abramovitch, Heterocyclic Compounds, Pyridine and its Derivate Vol 14, Part 1 - 4, Ed: A. Weissberger, E.C. Taylor und dort zitierte Literatur).

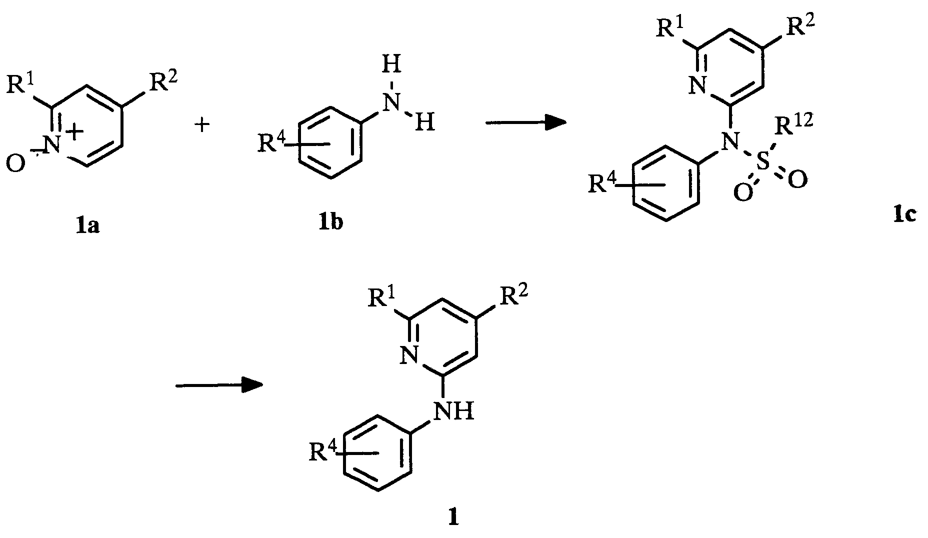

Alternativ dazu können Verbindungen der Formel I dadurch erhalten werden, daß man die Halogenbenzole Id unter Baseneinwirkung mit 2-Anilinopyridinen Ie zur Reaktion bringt. Als Basen eignen sich beispielsweise Alkalimetallhydride, z.B. Natrium-, Kaliumhydrid, Alkalimetallamide, z.B. Natriumamid oder Kaliumamid.

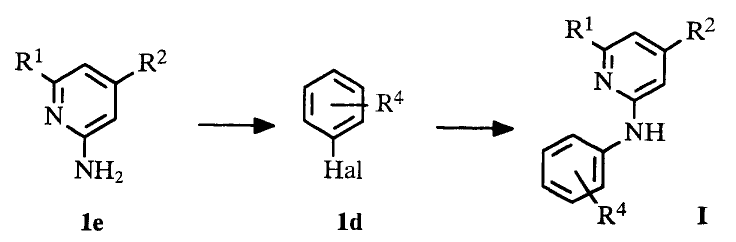

Des weiteren können Verbindungen der allgemeinen Formel I, dadurch hergestellt werden, daß man die Aniline If mit den Pyridinderivaten Ig mit oder ohne Basenzusatz zur Reaktion bringt (vgl. z.B. D. M. Bailaey et al., J. of. Med. Chem., 14 (1971) 439).

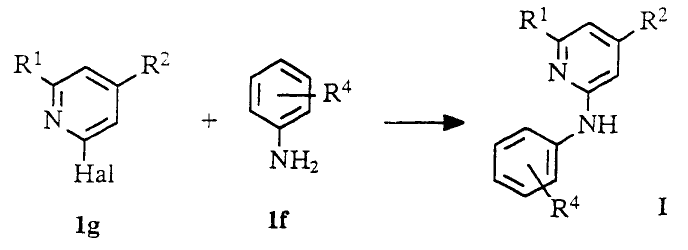

Eine weitere Variante besteht darin die Pyridine Ih mit α-Halogen-α-phenylhydrazonoessigsäureester Ii, bevorzugt α-Chlor-α-phenylhydrazonoessigsäure-t-butylester, zur Reaktion zu bringen. Die Hydrolyse der entstandenen N-Cyano-anilinopyridine Ij unter Basenzusatz liefert die Anilinopyridins I (R. Fusco et al. Gazz. chim. ital., **98** (1968) 511).

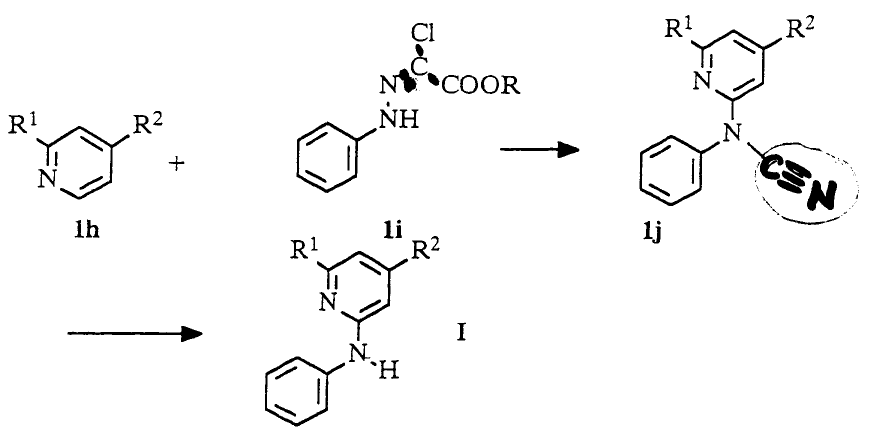

Verbindungen der allgemeinen Formel 3 können beispielsweise hergestellt werden indem man Verbindungen der allgemeinen Formel 1 mit R¹= CH₃ nach bekannten Verfahren mit Oxidationsmitteln wie z.B. Wasserstoffperoxid oder Persäuren bevorzugt m-Chlorperbenzoesäure, in protischen Lösungsmitteln, bevorzugt Essigsäure, oder in aprotischen Lösungsmitteln, z.B. Dichlormethan in die N-Oxide 2 überführt, die durch Umsetzung mit Alkylcarbonsäureanhydriden z.B. Essigsäureanhydrid und anschließender basischer Hydrolyse der Acylzwischenstufe die Hydroxymethylenverbindung 3 ergeben (siehe z.B. D. Bailey et al., J. of. Med. Chem., 14 (1971) 439).

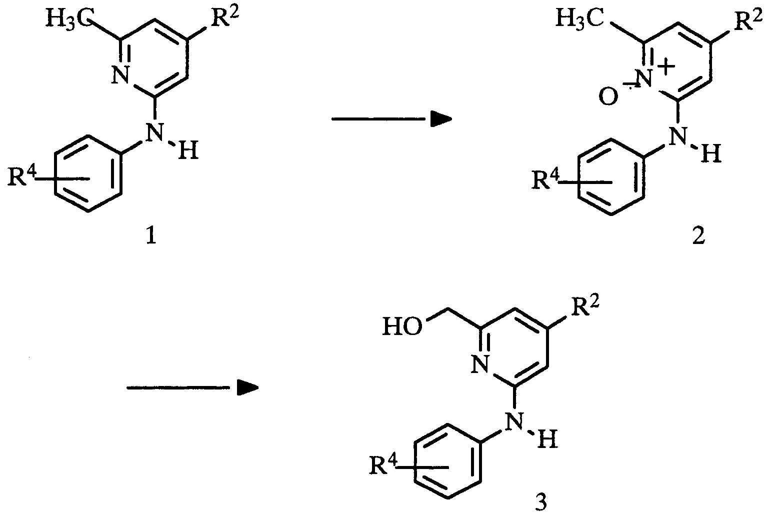

Verbindungen der allgemeinen Struktur **4**, wobei Hal für Fluor, Chlor, Brom, Jod steht, sind aus den entsprechenden Hydroxidverbindungen 3 durch literaturbekannte Verfahren zugänglich (z.B. Houben-Weyl, Methoden der Organischen Chemie, 4. Auflage Band 5/3 und 4, Halogenverbindungen).

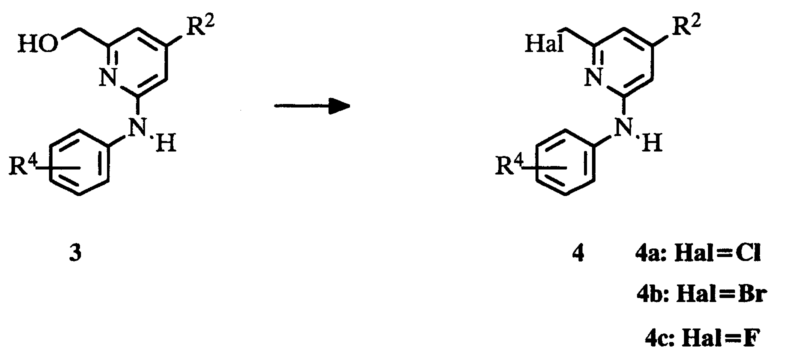

Als bevorzugte Verfahren zur Herstellung der Halogenide (Cl, Br) 4a,b kommen dabei die Umsetzung der Hydroxymethylenverbindung 4 mit geeigneten Halogenierungsmittel z.B. Thionylchlorid, Thionylbromid oder mit Halogenverbindungen des drei- oder fünfwertigen Phosphors (z.B. PBr₃ PCl₃) in Gegenwart oder Abwesenheit eines Protonenakzeptors in Gegenwart eines inerten Lösungsmittel z.B. Kohlenwasserstoffe wie Petrolether, Cyclohexan, Benzol, Toluol, Xylol in Frage. Die Reaktion wird bei Temperaturen, die zwischen -20°C und dem Siedepunkt des entsprechenden Lösungsmittels liegt, mit oder ohne Katalysator durchgeführt. Als Katalysatoren eignen sich beispielsweise DMF oder tertiäre Amine wie Triethylamin, N,N-Dimethylanilin oder Piperidin.

Zur Herstellung der Fluoride 4c werden die Hydroxymethylenverbindungen 3 mit einem Fluorierungsmittel, z.B. Diethylaminoschwefeltrifluorid in einem inerten Lösungsmittel z.B. Dichlormethan bei Raumtemperatur umgesetzt.

Verbindungen der allgemeinen Struktur 5, worin Alkyl-X für C₁-C₆-Alkyloxy (z.B. -CH₂-O-Alkyl wobei Alkyl z.B. für Methyl, Ethyl, Isopropyl steht) oder C₁-C₆-Alkylthiol (z.B. CH₂-S-Alkyl wobei Alkyl für z.B. Methyl, Ethyl, Isopropyl steht, können dadurch erhalten werden, daß die Verbindungen 4 mit einem Alkohol (z.B. Methanol, Ethanol, Isopropanol) vorzugsweise in einem inerten Lösungsmittel oder in einem Überschuß des betreffenden Alkohols der gleichzeitig als Verdünnungsmittel dient, in Gegenwart einer Base (z.B. Alkalimetalle, -hydroxide, -hydride) in einem Temperaturbereich der zwischen -20°C und dem Siedepunkt des entsprechenden Lösungsmittels liegt, zur Reaktion gebracht werden.

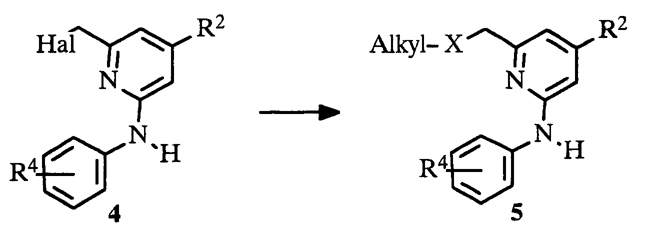

Verbindungen der allgemeinen Formel 6 können ausgehend von den Hydroxymethylenverbindungen 3 durch allgemein bekannte Verfahren hergestellt werden (siehe z.B. Houben-Weyl Methoden der Organischen Chemie, 4. Auflage, E3, 265 ff).

Als bevorzugtes Verfahren kommt die Oxidation mit Dimethylsulfoxid in Gegenwart geeigneter Hilfsreagenzien z.B. Oxalylchlorid/Triethylamin zur Anwendung. Als Verdünnungsmittel kommen inerte organische Lösungsmittel z.B. Kohlenwasserstoffe wie Petrolether, Cyclohexan, Benzol oder chlorierte Kohlenwasserstoffe wie Dichlormethan, Chloroform, 1,2-Dichlorethan oder Ether wie z.B. Diethylether, Tetrahydrofuran, Dioxin infrage. Die Reaktion wird in einem Temperaturbereich zwischen -80°C und 50°C bevorzugt -70°C bis -10°C durchgeführt.

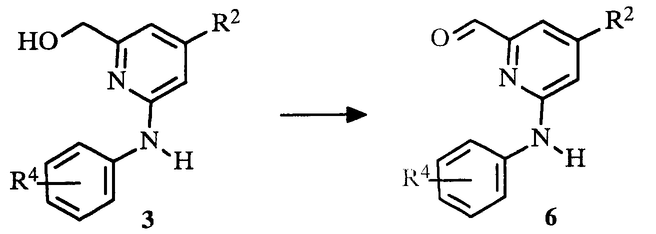

Die Oximether der allgemeinen Formel 7 sind nach an sich bekannten Verfahren zugänglich (siehe Houben-Weyl, Methoden der Organischen Chemie, 4. Auflage, Band 10/4 S.55 ff). Als bevorzugtes Verfahren kommt die Umsetzung der Aldehyde der allgemeinen Formel 6 mit einem Hydroxylamin H₂N-O-R⁵ oder dem entsprechenden Hydrochlorid (z.B. H, Methyl, Ethyl, Hydroxyethyl, Halogenethyl, Benzyl oder durch Fluor, Chlor oder Methyl substituiertes Benzyl, C₃-C₄-Alkenyl, C₃-C₄-Alkinyl, CH₂Ph) in einem geeigneten Verdünnungsmittel und geeigneter Hilfsreagenzien zur Anwendung.

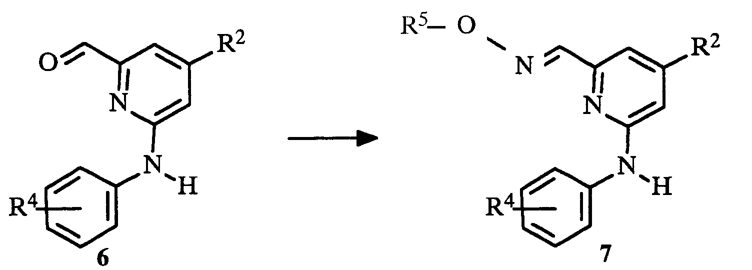

Als Verdünnungsmittel kommen Lösungsmittel wie chlorierte Kohlenwasserstoffe z.B. Dichlormethan, 1,2-Dichlorethan oder Alkohole, wie z.B. Methanol, Ethanol, Isopropanol oder Nitrile wie Acetonitril, Propionitril und Wasser infrage. Als Reaktionshilfsmittel kommen anorganische und organische Basen infrage z.B. Alkalihydroxide, wie z.B. Natriumhydroxid, oder Alkalialkoholate, wie z.B. Natriummethylat oder Alkalicarbonate wie z.B. Natrium- und Kaliumcarbonat, tertiäre Amine: Triethylamin infrage. Die Reaktion wird bei Temperaturen von 0°C bis zum Siedepunkt des eingesetzten Verdünnungsmittels vorzugsweise 20°C - 80°C, durchgeführt.

Verbindungen der allgemeinen Formel 8 (R⁶ z.B. C₁-C₆-Alkyl, bevorzugt Methyl, Ethyl, Propyl oder C₃-C₆-Cycloalkyl z.B. Cyclopropyl, Cyclopentyl, Cyclohexyl oder durch Chlor, Hydroxy, Cyclopropyl, oder Benzyl substituiertes C₁-C₂-Alkyl, oder Allyl, Propargyl ferner Phenyl oder durch Fluor, Chlor Methyl, Trifluormethyl, substituiertes Phenyl) können hergestellt werden, indem man eine Verbindung der allgemeinen Formel 6 mit einem primären Amin der Formel H₂N-R⁶, worin R⁶ die oben beschriebene Bedeutung hat in einem inerten lösungsmittel z.B. Kohlenwasserstoffe: Petrolether, Cyclohexan, bevorzugt Toluol, Benzol, Xylol, in Gegenwart eines Reaktionshilfstoff (z.B. geringe Mengen anorganischer oder organischer Säuren z.B. Toluolsulfonsäure), bei Temperaturen zwischen 0°C und dem Siedepunkt des jeweiligen Verdünnungsmittels, bevorzugt 80 bis 120°C umsetzt.

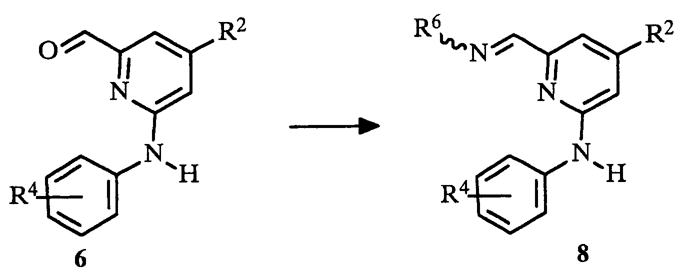

Verbindungen der allgemeinen Formel 9 können durch Hydrierung der Verbindungen 8 mit elementarem Wasserstoff unter Normaldruck oder mit Überdruck in Gegenwart eines Katalysators, z.B. Palladium-Kohle oder Raney-nickel in einem inerten Lösungsmittel, z.B. Ethylacetat, Tetrahydofuran, oder Dioxin bei Temperaturen von 10°C - 120°C, bevorzugt 20°C - 60°C, hergestellt werden (siehe z.B. Houben-Weyl, Methoden der Organischen Chemie, Band 11/1 S.602 ff).

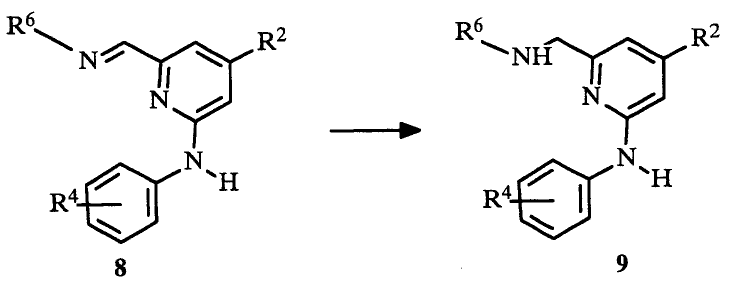

Verbindungen der allgemeinen Form 10 mit R¹ = CN, können aus Verbindungen der allgemeinen Form 7 mit R⁵ = H (7a), nach an sich bekannten Verfahren hergestellt werden (siehe z.B. Houben-Weyl, Methoden der organischen Chemie, 4. Auflage, Band E5 S. 1346), wobei die Aldoxime in Gegenwart eines Lösungsmittel (z.B. Acetonitril, Dichlormethan) mit einem Dehydratisierungsmittel (z.B. Acetanhydrid, Trichlormethylmonochlorkohlensäureester, Trichloracethylchlorid, SOCl₂ u. a.) bei Temperaturen zwischen -20°C - 50°C, bevorzugt 0°C - 20°C zur Reaktion gebracht werden.

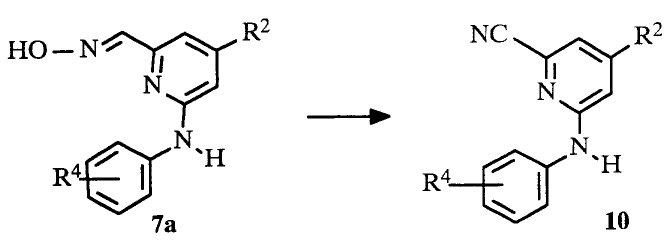

Verbindungen der allgemeinen Formel Ij können einerseits wie oben beschrieben ausgehend von 2,4-disubstituierten Pyridinen Ih, durch Umsetzung mit α-Chlor-α-phenylhydrazonoessigsäureester erhalten werden. Alternativ dazu können die Verbindungen ausgehend von Verbindungen der allgemeinen Formel 1, in der die Substituenten, die im Anspruch 1 beschriebenen Bedeutungen haben und R³ für Wasserstoff steht, durch Umsetzung mit Bromcyan in einem inerten Lösungsmittel z.B. Diethylether, Tetrahydrofuran, Dimethylformamid, Dimethylsulfoxid in Gegenwart einer organischen oder anorganischen Base erhalten werden (siehe z.B. S. Salman et al., Indian J. of. Chem., 27B (1988) 583).

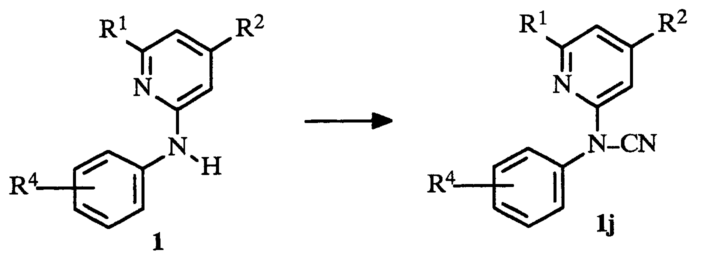

Verbindungen der allgemeinen Formel 12 (R⁹ z.B. H, C₁-C₆-Alkyl (z.B. Methyl, Ethyl, Isopropyl), C₁-C₆-Halogenalkyl (z.B. Trifluormethan, Trichlormethan)) können nach an sich bekannten Verfahren hergestellt werden indem man Verbindungen der allgemeinen Formel 1, worin für R³ = H steht, mit einem Carbonsäurederivat umsetzt. Für die Herstellung der erfindungsgemäßen Verbindungen geeignete Carbonsäurenderivate sind z.B. Säurechloride, Säureanhydride oder Orthocarbonsäureester. Die Acylierung der Verbindungen der allgemeinen Formel 1 mit R³ = H, kann mit oder ohne Lösungsmittel erfolgen, wobei das Lösungsmittel zugleich (z.B. Pyridin) als säurebindendes Mittel dienen kann. Als säurebindendes Mittel kann auch das Ausgangsprodukt selbst, andere organische Basen (z.B. Triethylamin) oder anorganische Basen dienen.

Für die Acylierungsreaktion eignen sich alle üblichen Lösungsmittel (z.B. Pyridin, Dichlormethan, Dioxan, Tetrahydrofuran).

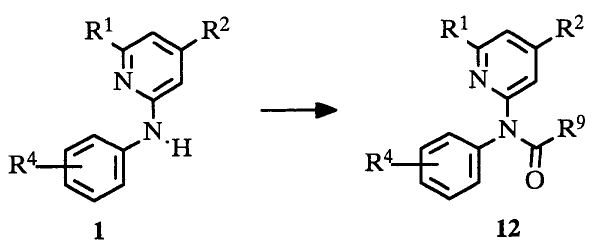

Verbindungen der allgemeinen Form mit 14, wobei Hal z.B. für Chlor, Brom steht, können dadurch erhalten werden, daß man die N-Oxide 13 mit Phosphoroxyhalogeniden (Halogen bedeutet z.B. Cl, Br) in einem inerten Lösungsmittel oder bevorzugt ohne Lösungsmittel bei Temperaturen zwischen 20° und dem Siedepunkt des jeweiligen Lösungsmittels bevorzugt 70 - 120° umsetzt. (siehe z.B. R. A. Abramovitch, E. M. Smith; Pyridin-1-oxide, in Heterocyclic Compounds, Pyridine and its Derivate Vol 14, Part2, S. 1-261, Ed: A. Weissberger, E.C. Taylor).

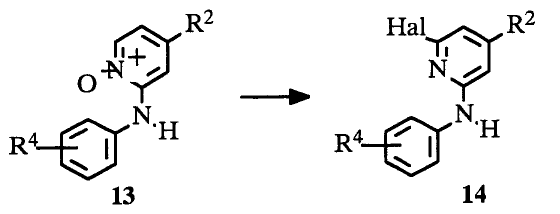

Verbindungen der allgemeinen Struktur 15, worin Alkyl-X für C₁-C₆-Alkyloxy (z.B. O-Alkyl wobei Alkyl z.B. für Methyl, Ethyl, Isopropyl steht) oder C₁-C₆-Alkylthio (z.B. S-Alkyl wobei Alkyl für z.B. Methyl, Ethyl, Isopropyl) steht, können dadurch erhalten werden, daß die Verbindungen 14 mit einem Alkohol oder einem Thiol der allgemeinen Form HX-Alkyl wobei X = O bzw S und Alkyl die oben beschriebenen Bedeutungen hat, (z.B. Methanol, Ethanol, Isopropanol) vorzugsweise in einem inerten Lösungsmittel oder in einem Überschuß des betreffenden Alkohols, der gleichzeitig als Verdünnungsmittel dient, in Gegenwart einer Base (z.B. Alkali-metalle, -hydroxide, -hydride) in einem Temperaturbereich der zwischen -20°C und dem Siedepunkt des entsprechenden Lösungsmittels liegt, zur Reaktion gebracht werden.

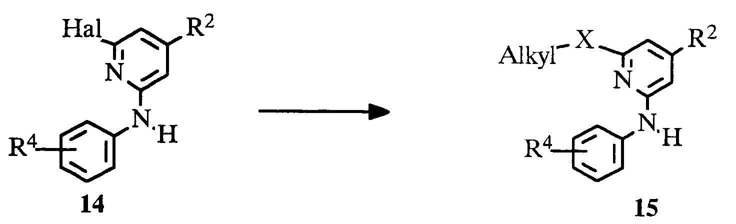

Die folgenden Beispiele erläutern die Herstellung der Verbindungen I.

### Beispiel 1

### 2-(N-Tosylsulfonyl)-anilino-6-cyclopropyl-4-methylpyridin (Verbindung Nr. 28)

a) Zu 5.7g( 0.041mol) 2-Cyclopropyl-4-methylpyridin in 50 ml Dichlormethan werden 7.74 g (0.045mol) m-Chlorperbenzoesäure in 125 Dichlormethan bei Raumtemperatur (20°C) getropft. Man beläßt 6h bei RT (Raumtemperatur), und gibt anschließend 3 g Natriumsulfit zu. Nach 1h wird die Reaktionsmischung zuerst mit gesättigter Natriumcarbonatlösung und anschließend mit Wasser gewaschen. Die Waschlösung wird mit Dichlormethan extrahiert, die organischen Phasen vereint, getrocknet und eingeengt. Es verbleiben 5.7g (98%) 2-Cyclopropyl-4-methyl-pyridin-N-oxid als schwach gelbgefärbtes Öl.
b) Zu 2g (0,0134 mol) des oben erhaltenen Öls, 1,25 g (0,0134 mol) Anilin und 6,48g (0,034mol) Toluol-4-sulfonylchlorid in 50 ml Dichlormethan werden bei RT 22ml einer 10 % NaOH Lösung (0.054mol) getropft. Man läßt 24h bei RT rühren, trennt die Phasen, extrahiert die wäßrige Phase mit Dichlormethan. Die vereinigten organischen Phasen werden über Natriumsulfat getrocknet und eingengt. Das verbleibende Öl wird säulenchromatographisch (Cyclohexan:Essigester = 2:1) gereinigt. Man erhält 3,54 g (70 %) der Titelverbindung als schwach gelb gefärbtes Öl.

### Beispiel 2

### 2-Anilino-6-cyclopropan-4-methylpyridin (Verbindung Nr. 24)

3,2g (0.0085 mol) 2-(N-p-Toluolsulfonyl)anilino-6-cyclopropyl-4-methylpyridin werden in 150 ml HClKonz. aufgenommen und 8 h unter Rückfluß erhitzt. Die Lösung wird anschließend weitgehend eingeengt, auf Wasser gegeben, mit Kaliumcarbonat neutralisiert und mit Dichlormethan extrahiert.

Nach dem Abdestillieren des Lösungsmittel verbleiben 1,64 g (91,8 %) der Titelverbindung.

### Beispiel 3

### 2-(N-Acetyl)anilino-4,6-dimethylpyridin (Verbindung Nr. 7)

Zu 1 g (0.005 mol) 2-Anilino-4,6-dimethylpyridin in 10 ml Eisessig werden bei RT 0,51 g (0,005 mol) Essigsäureanhydrid getropft. Man läßt 1h nachrühren, gießt auf Wasser, stellt mit 10 % NaOH neutral und extrahiert mit Dichlormethan. Die organischen Extrakte werden getrocknet und eingeent. Es verbleibt ein hellbraunes Öl, das alsbald kristallisiert (0,64 g; 53 %).

Die folgenden Verbindungen sind beispielsweise zur Anwendung gemäß dem beanspruchten Verfahren zur Bekämpfung von Schädlingen geeignet.

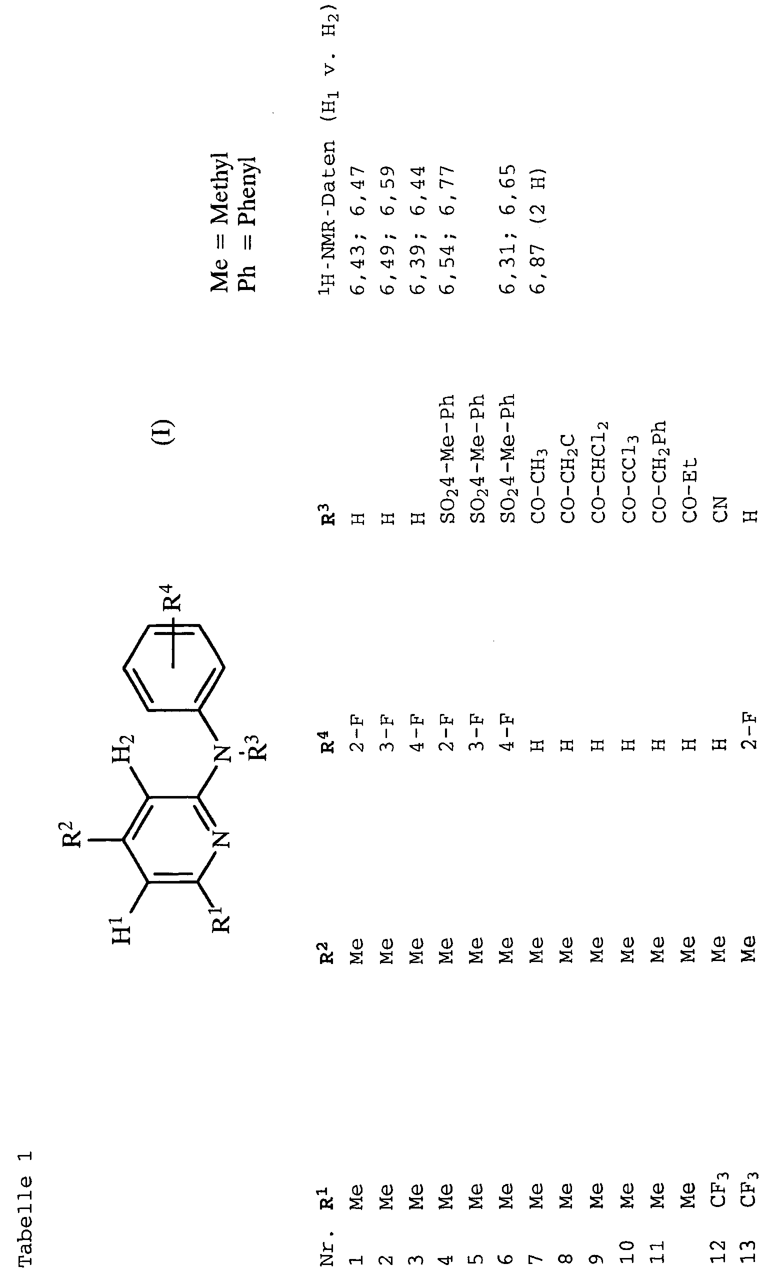

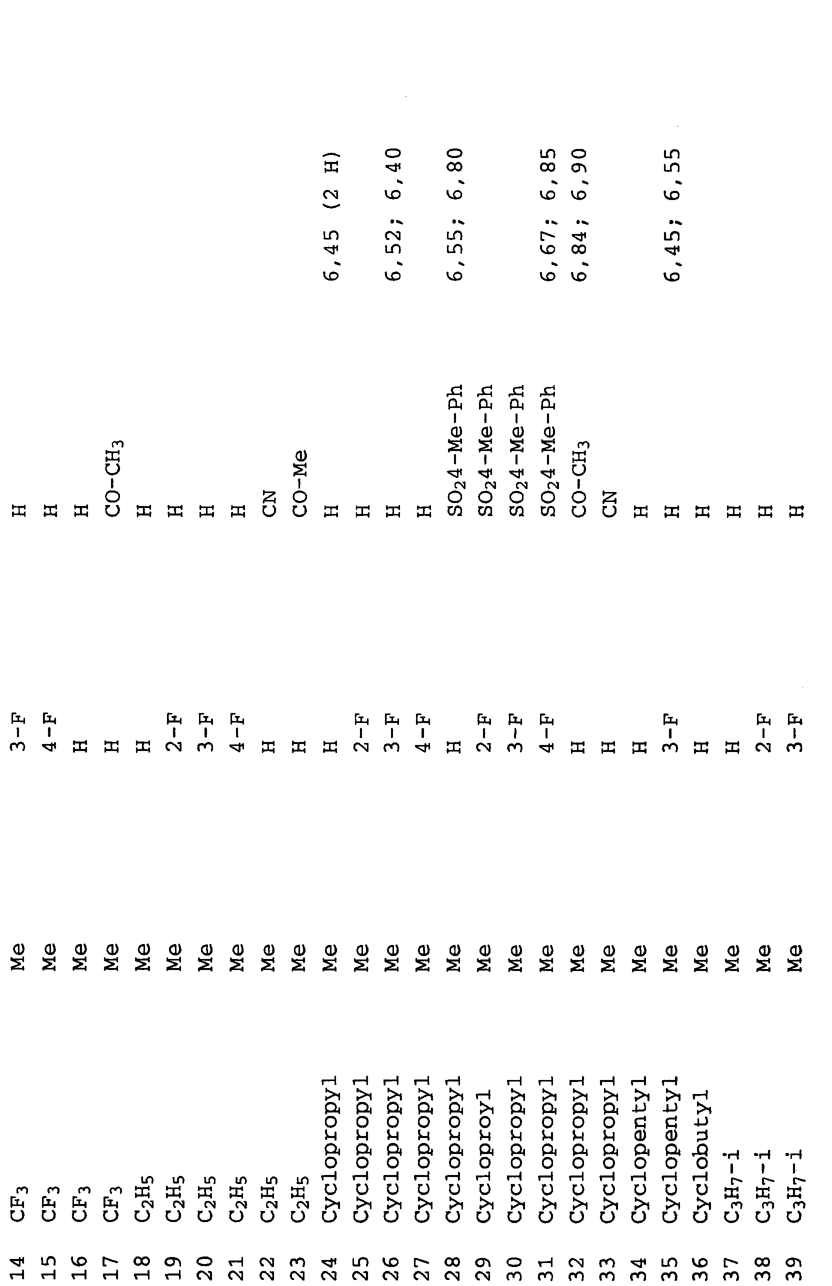

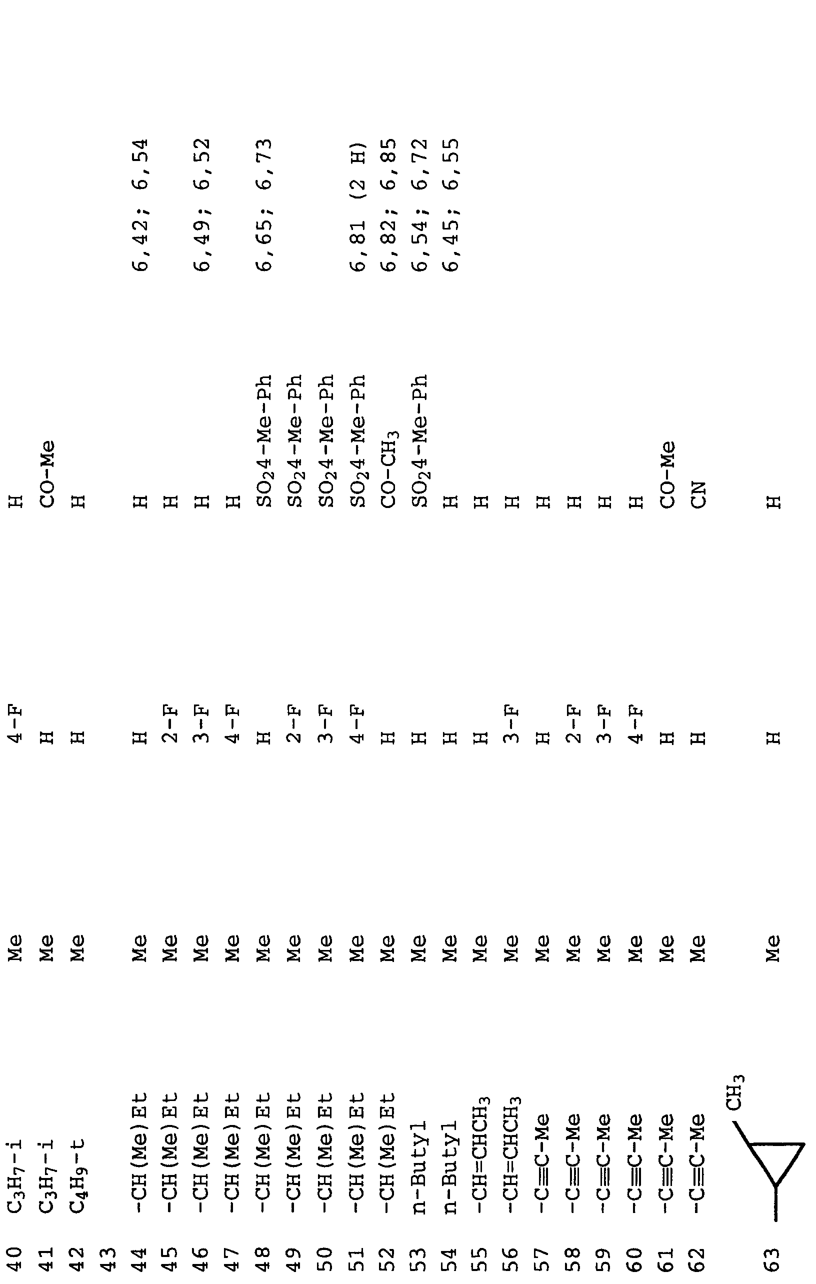

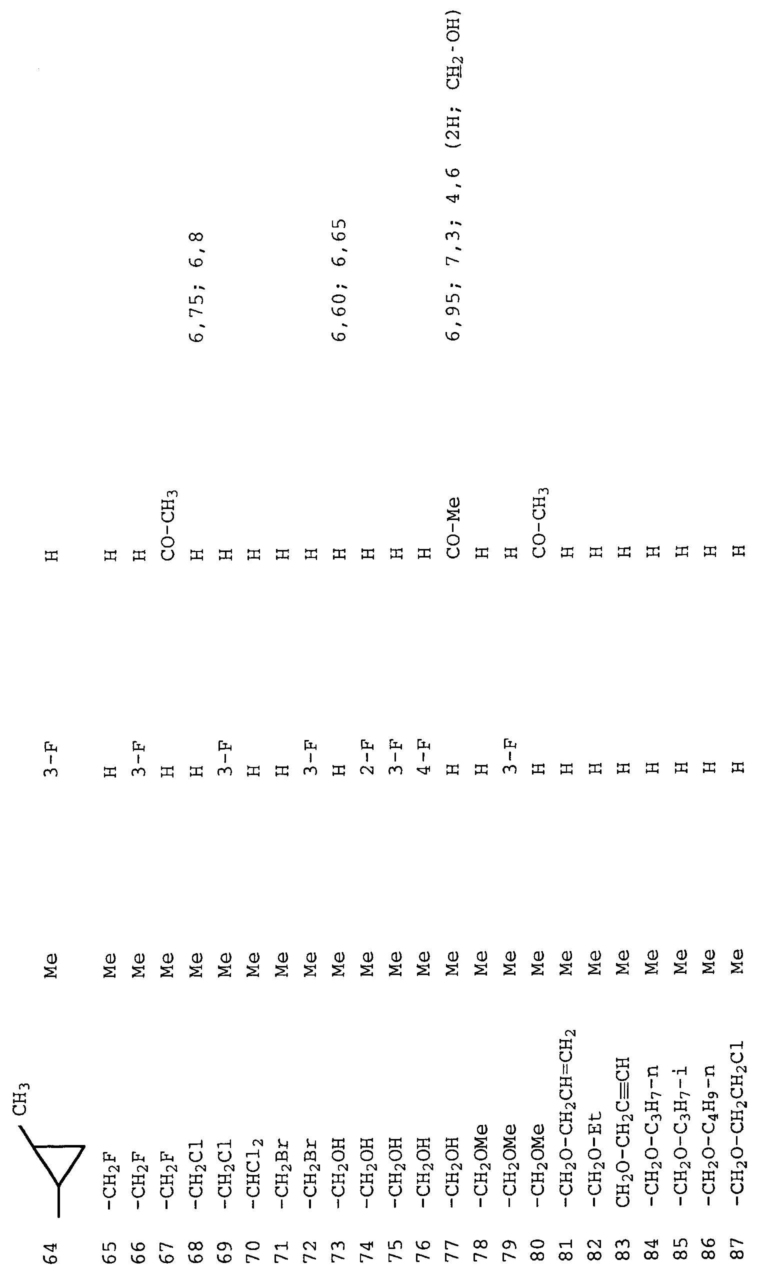

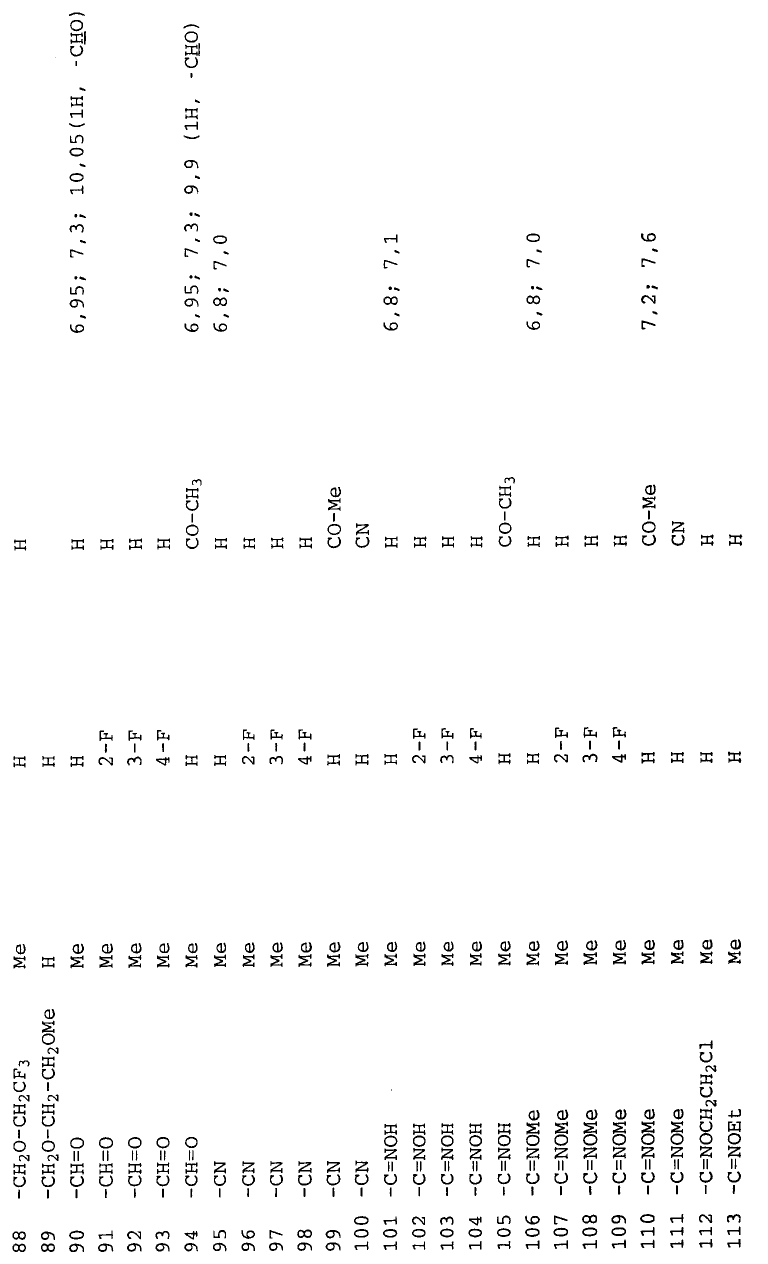

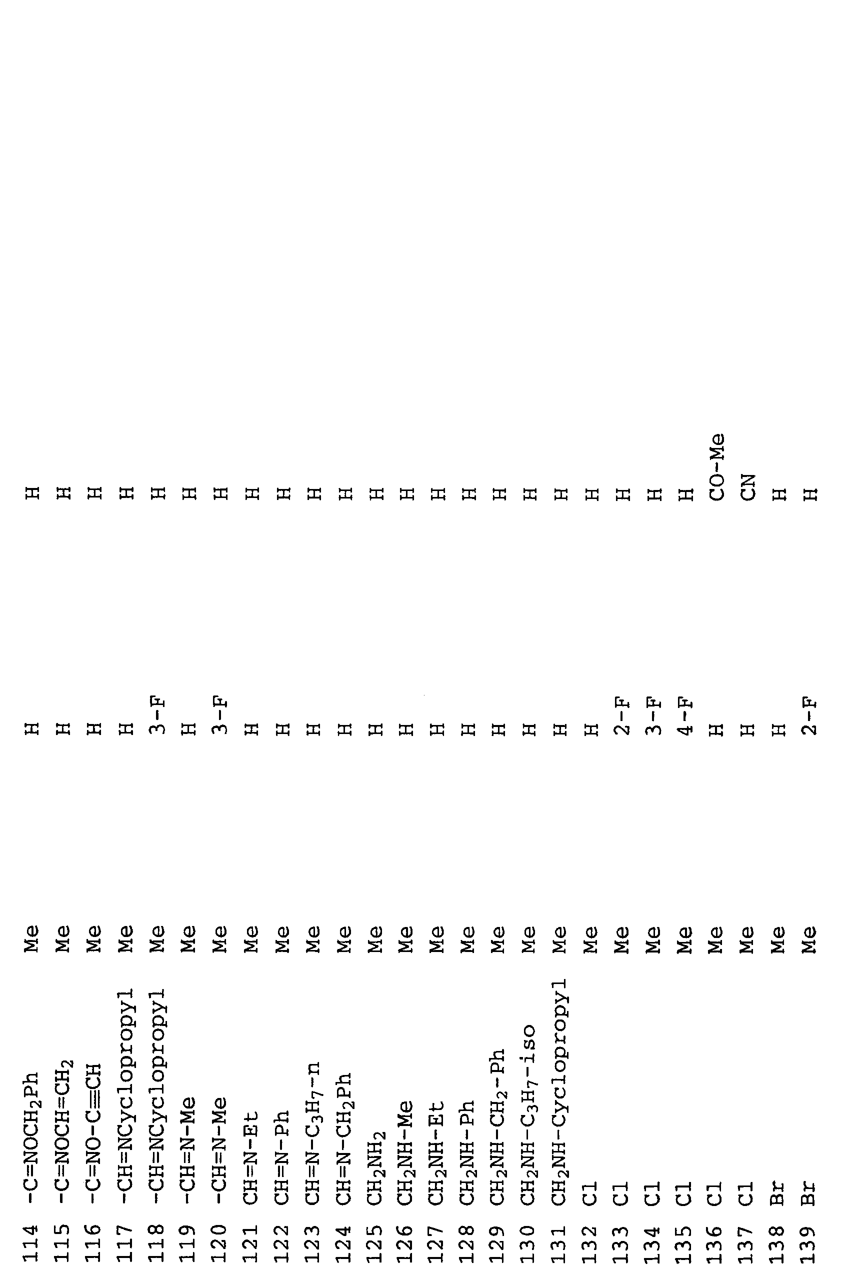

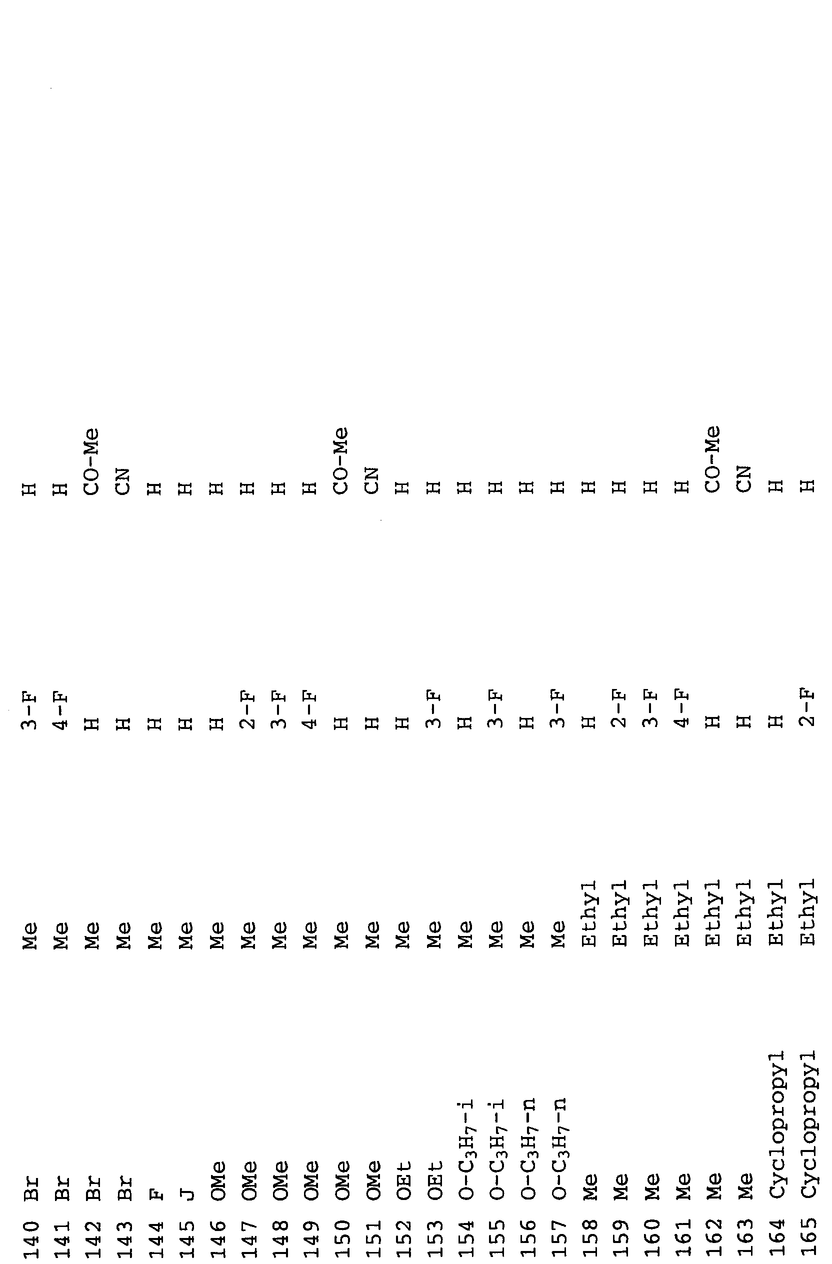

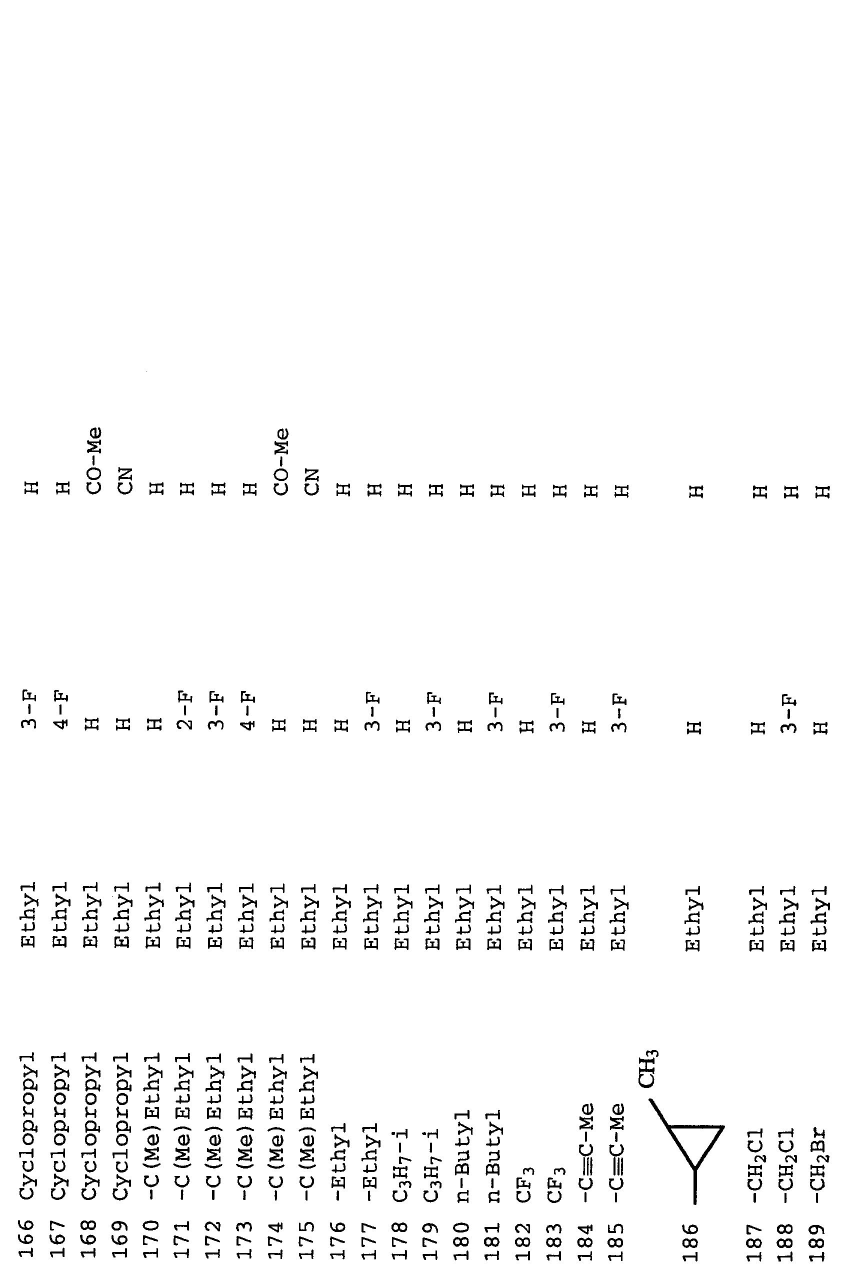

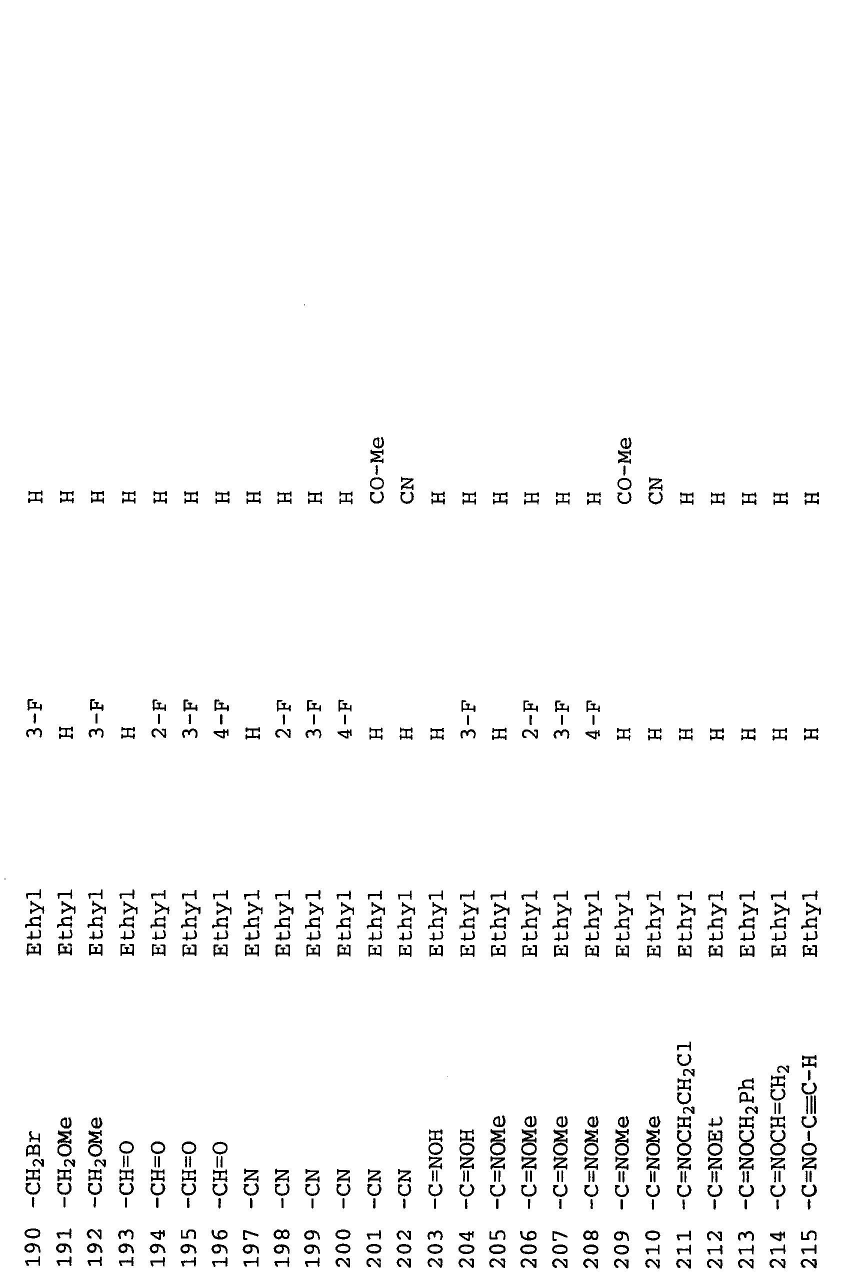

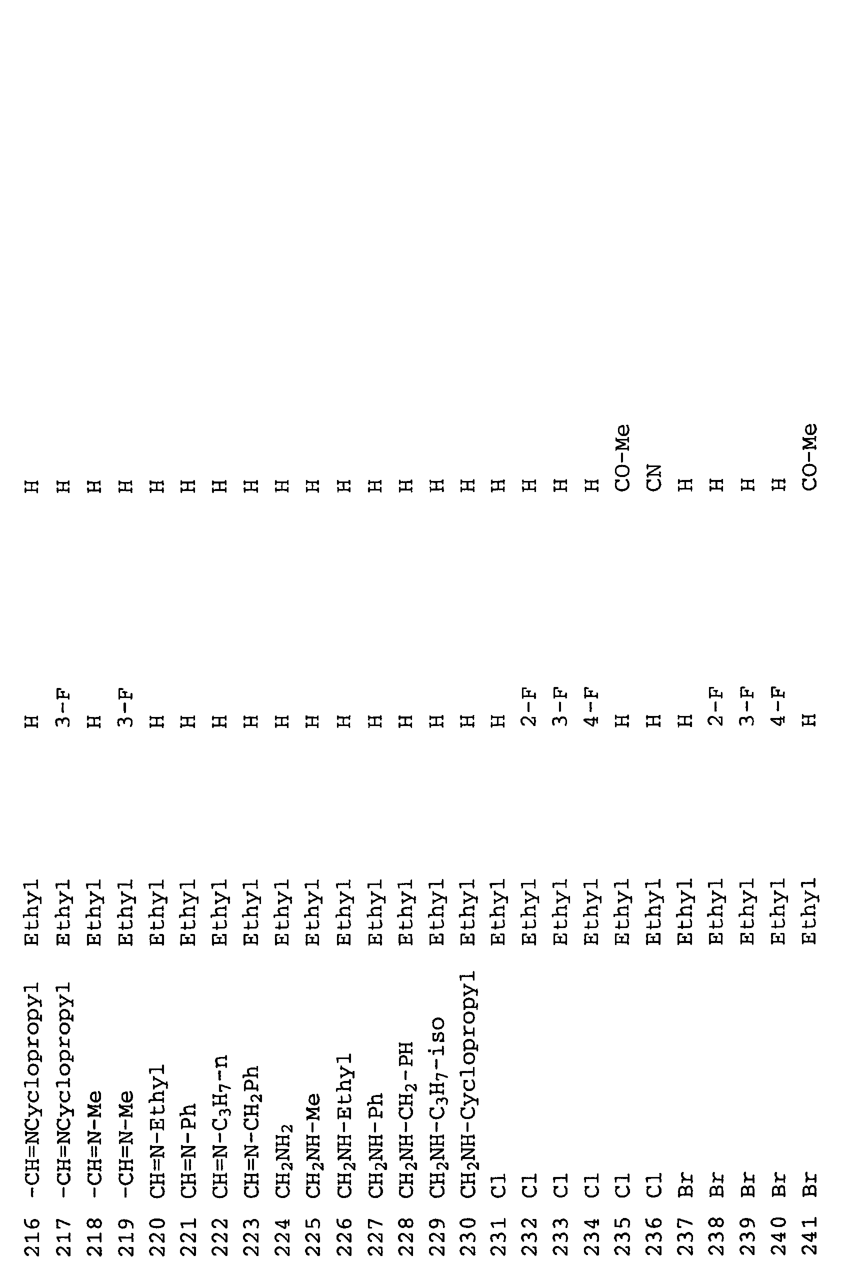

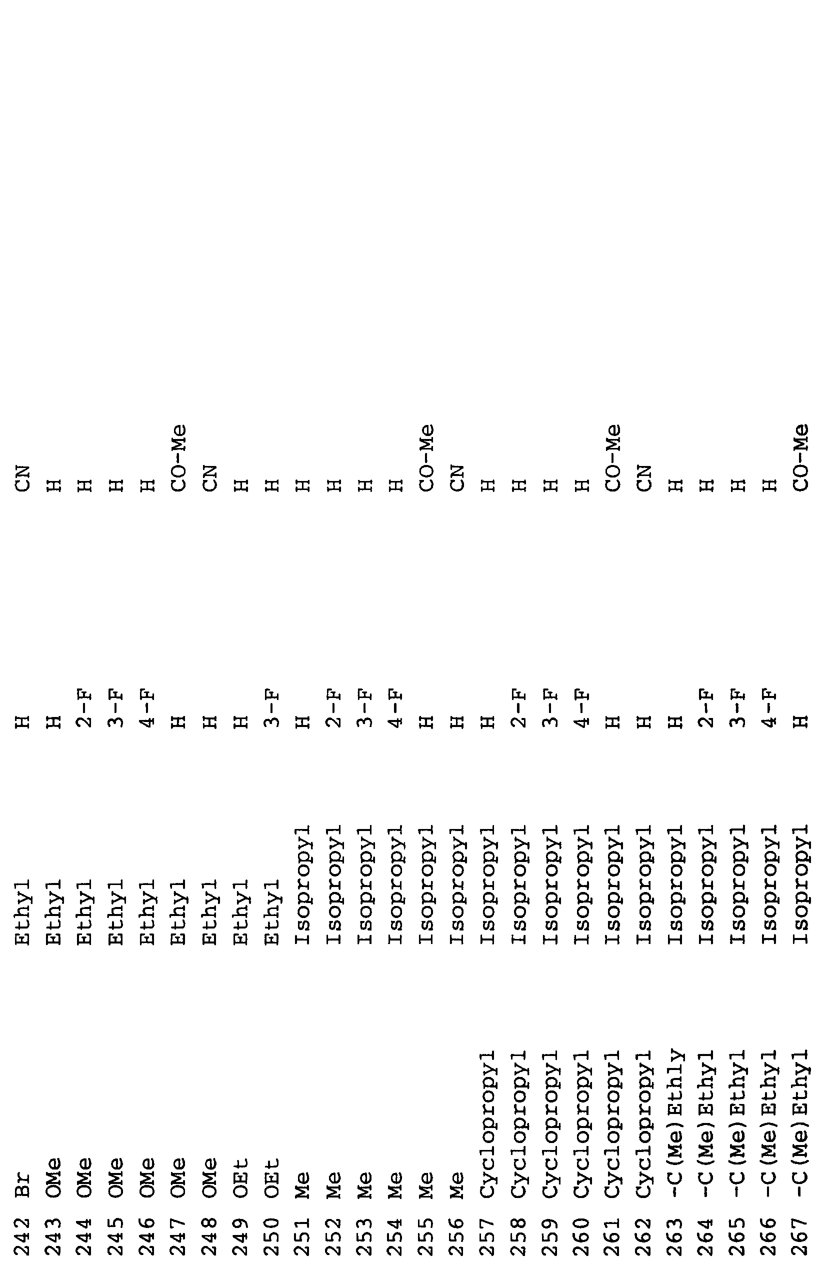

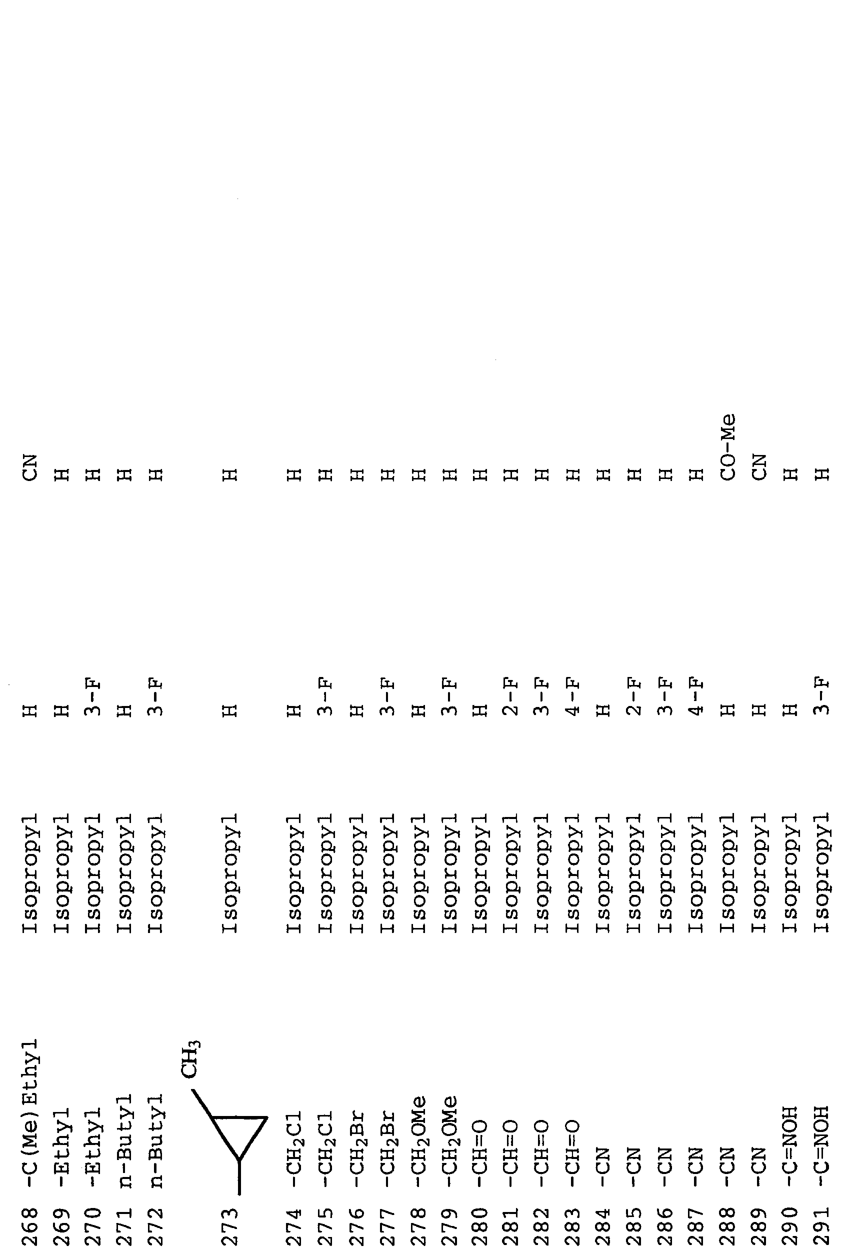

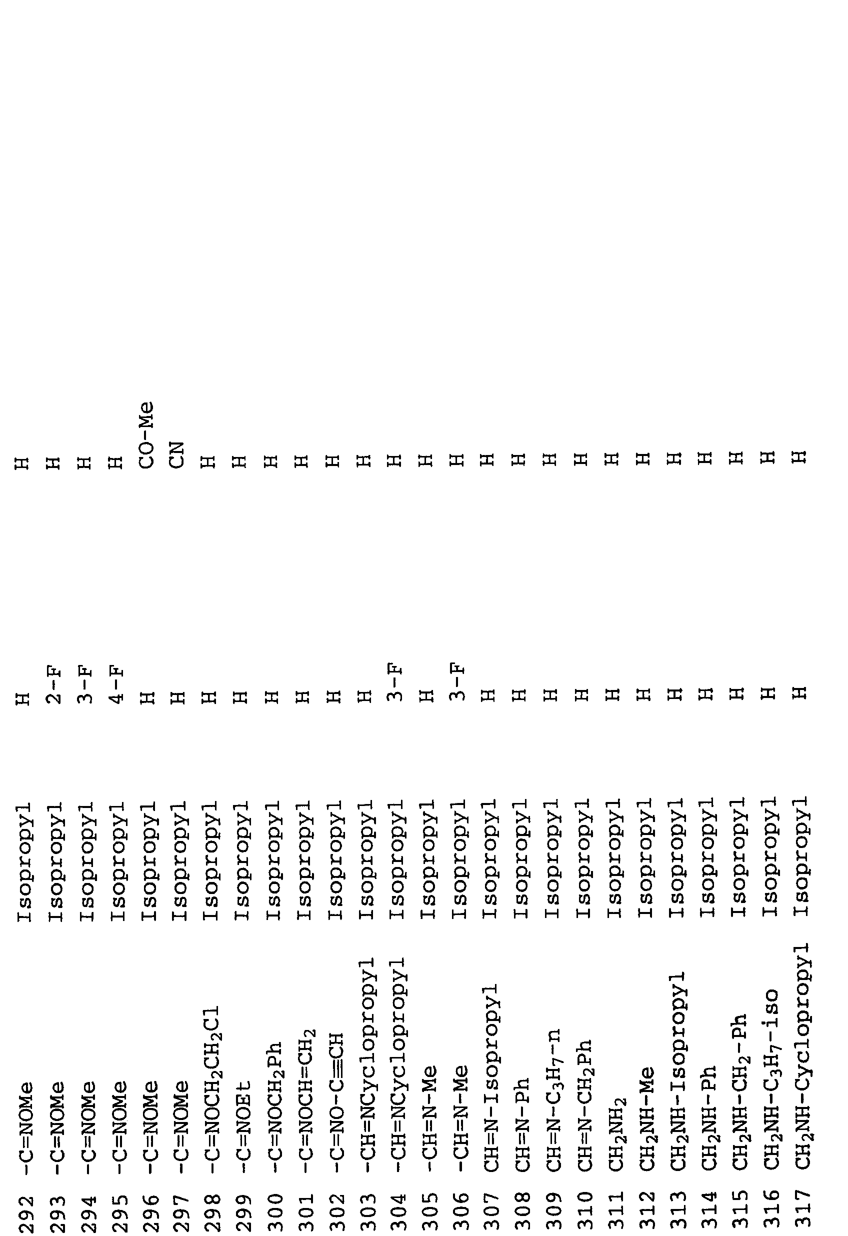

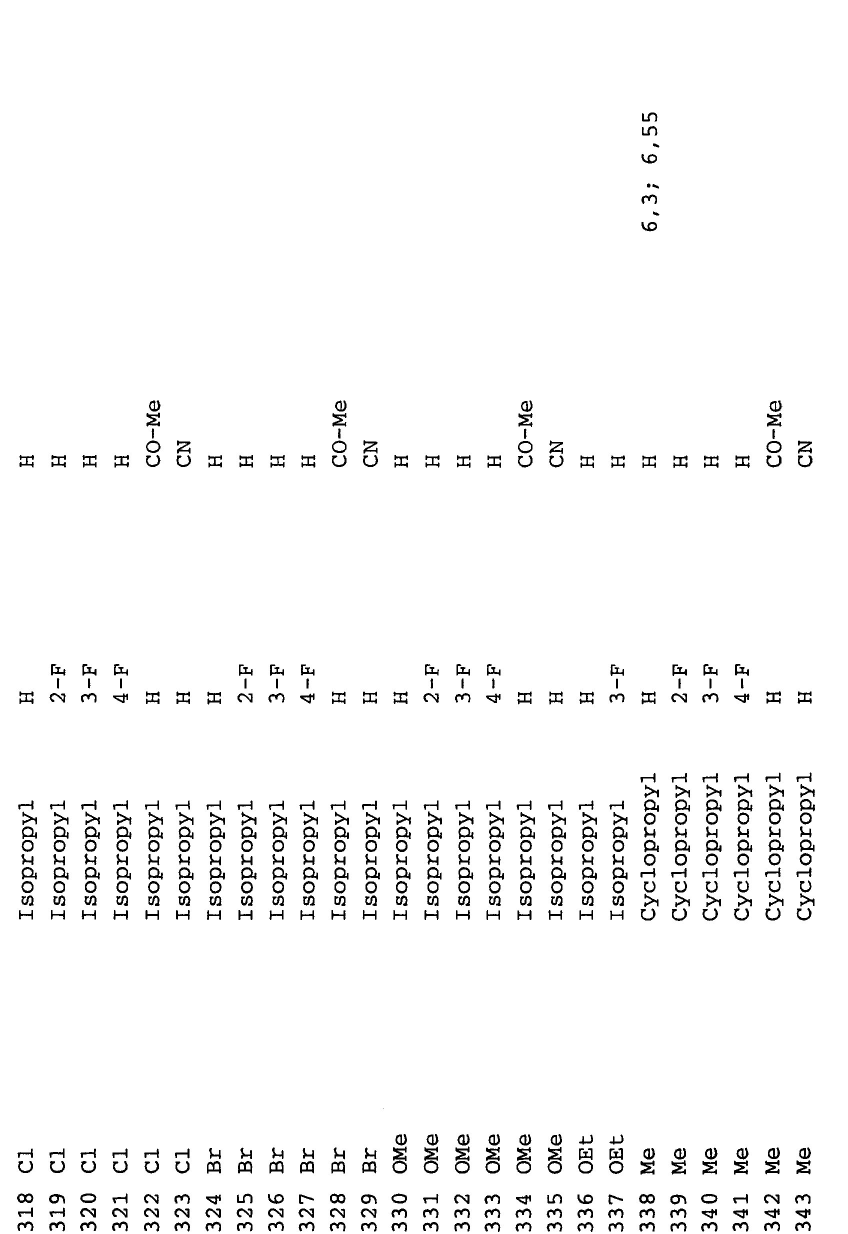

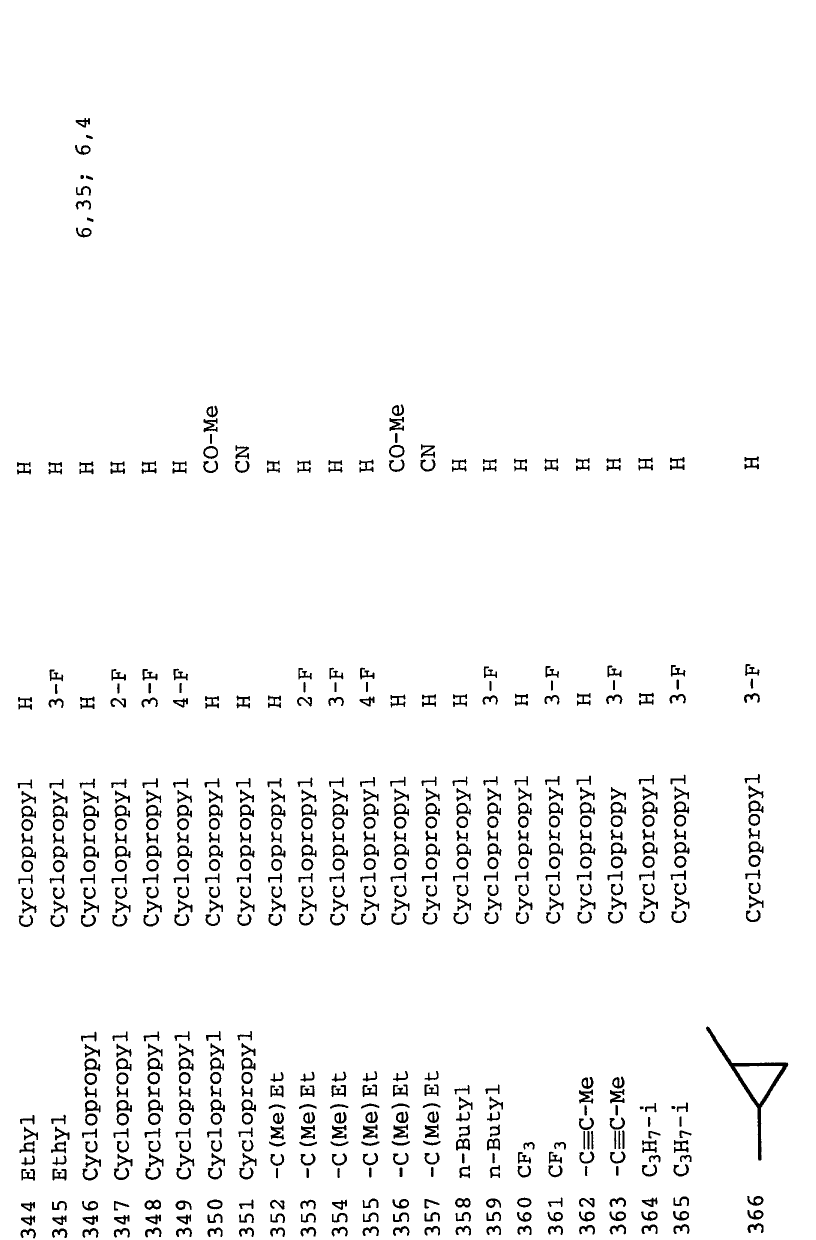

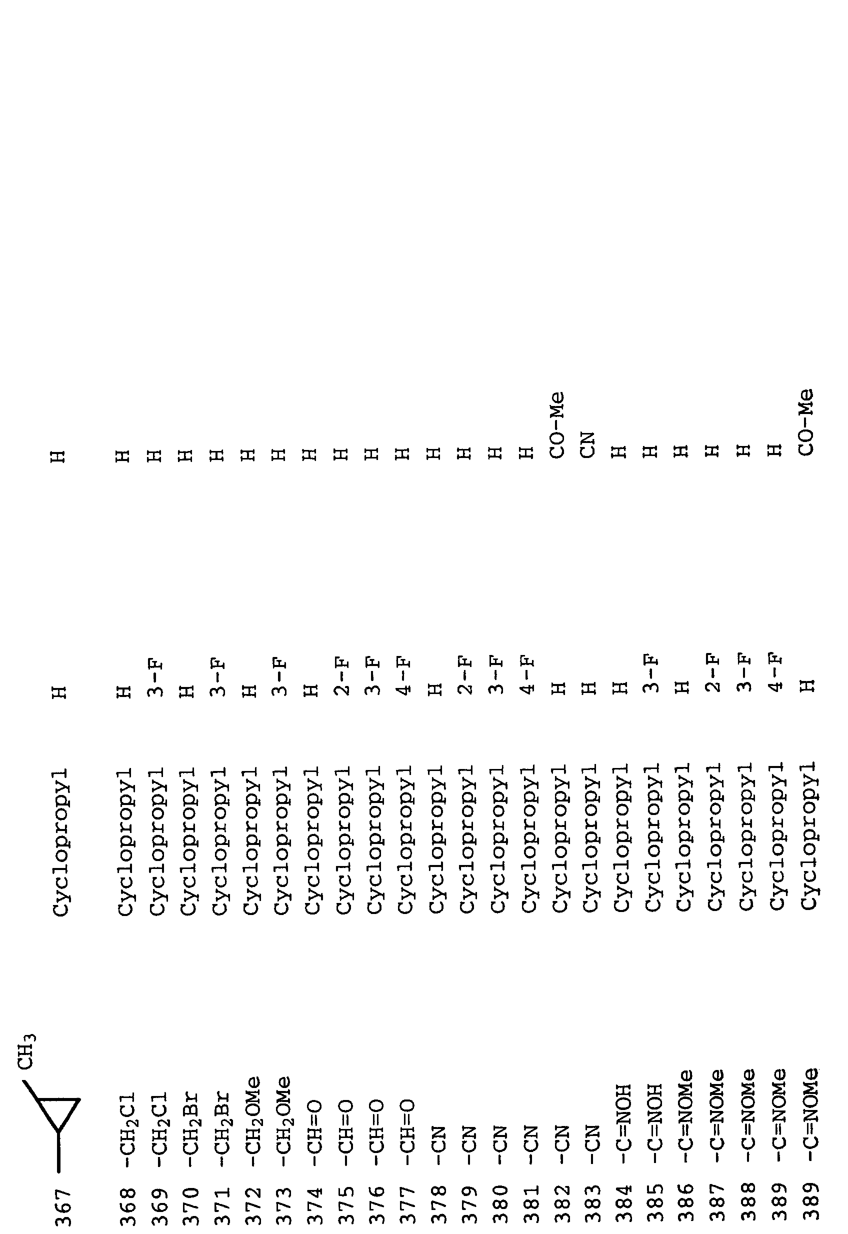

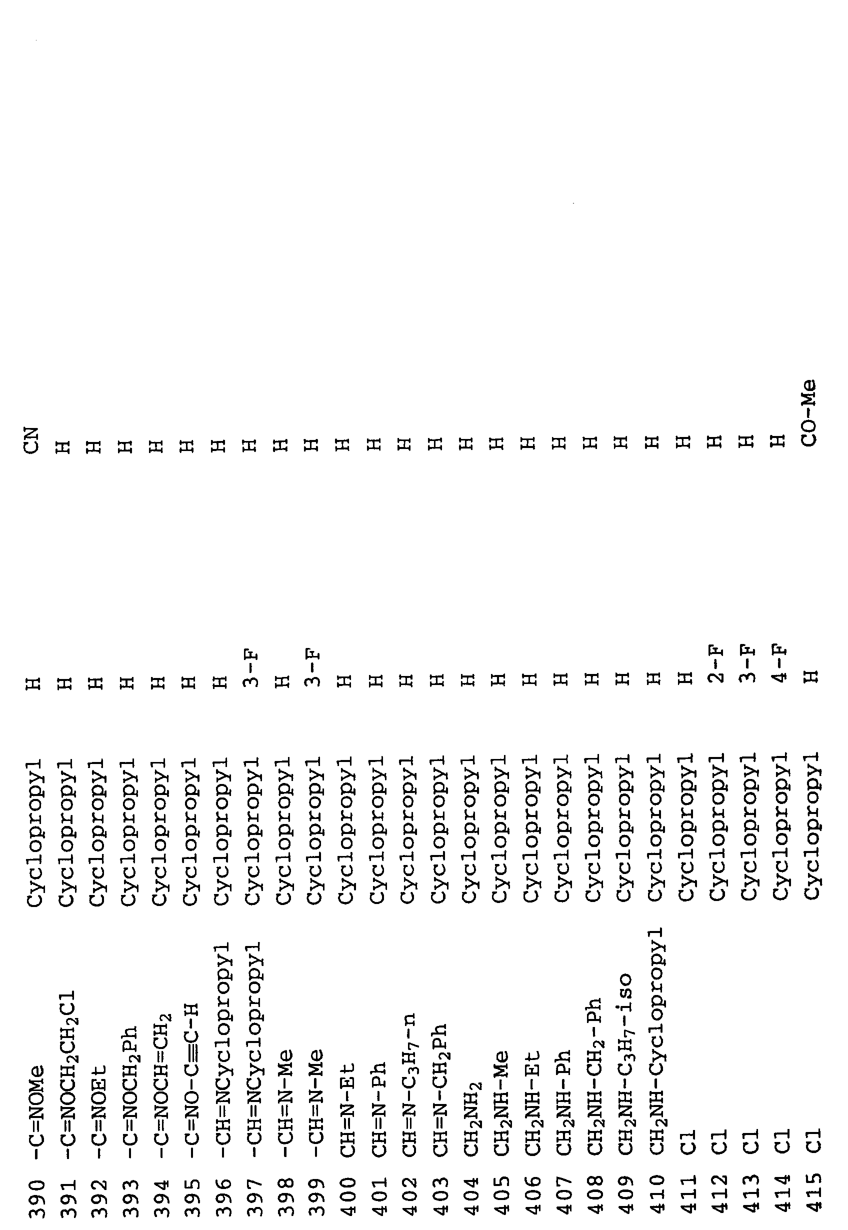

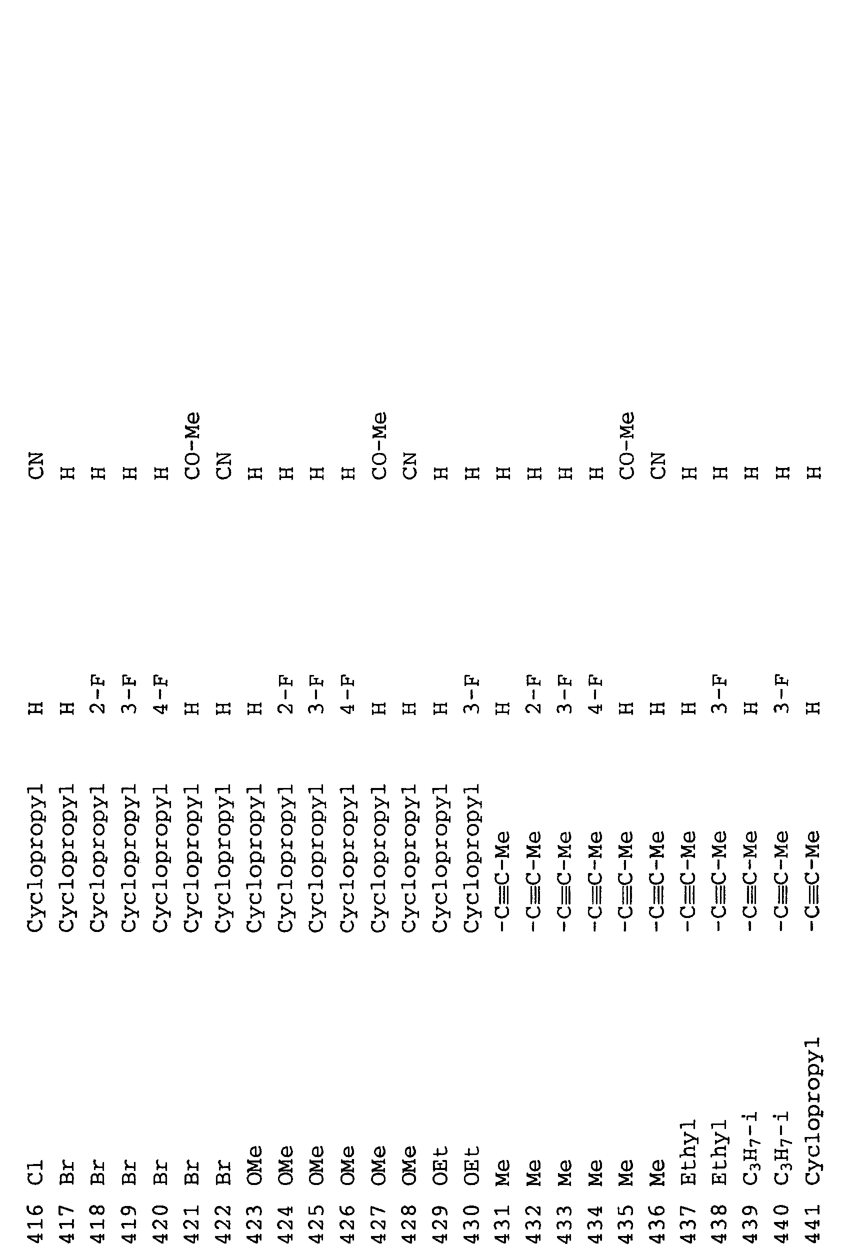

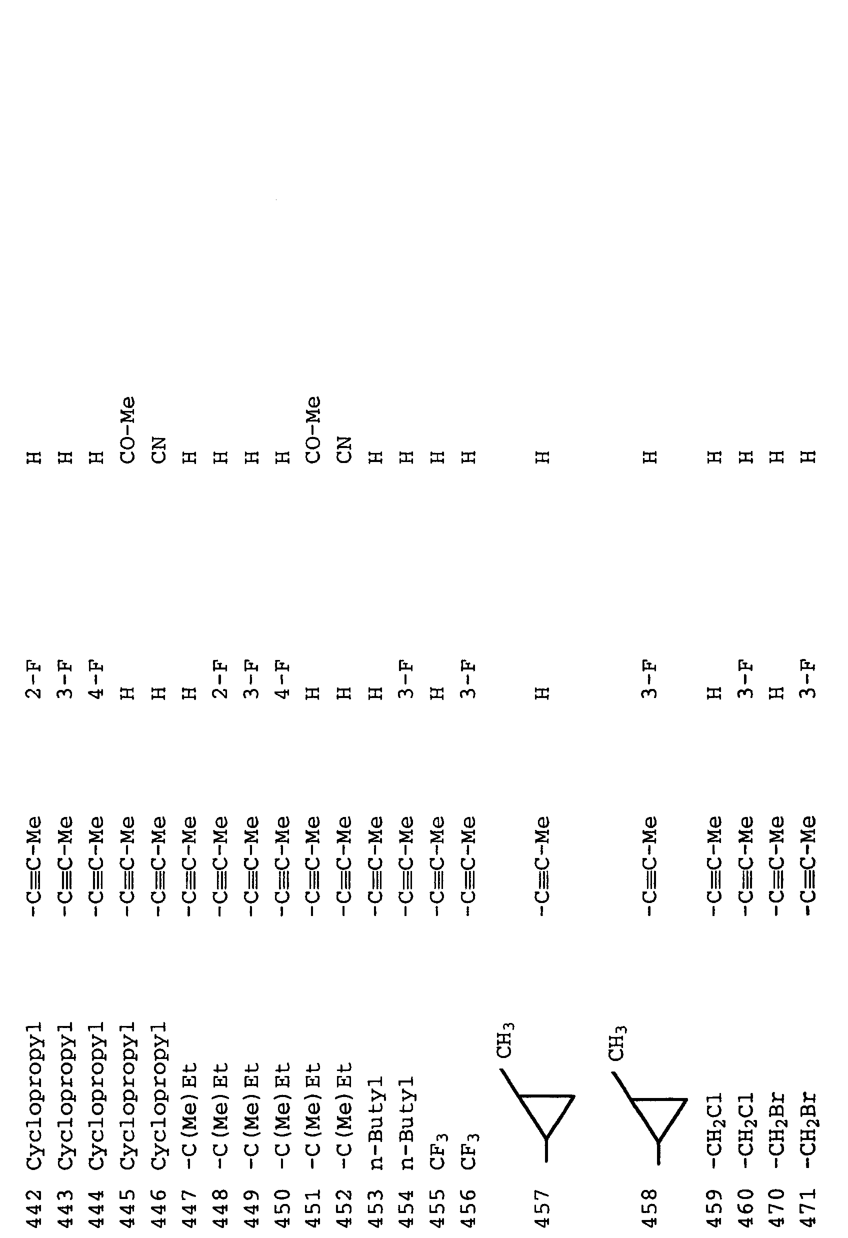

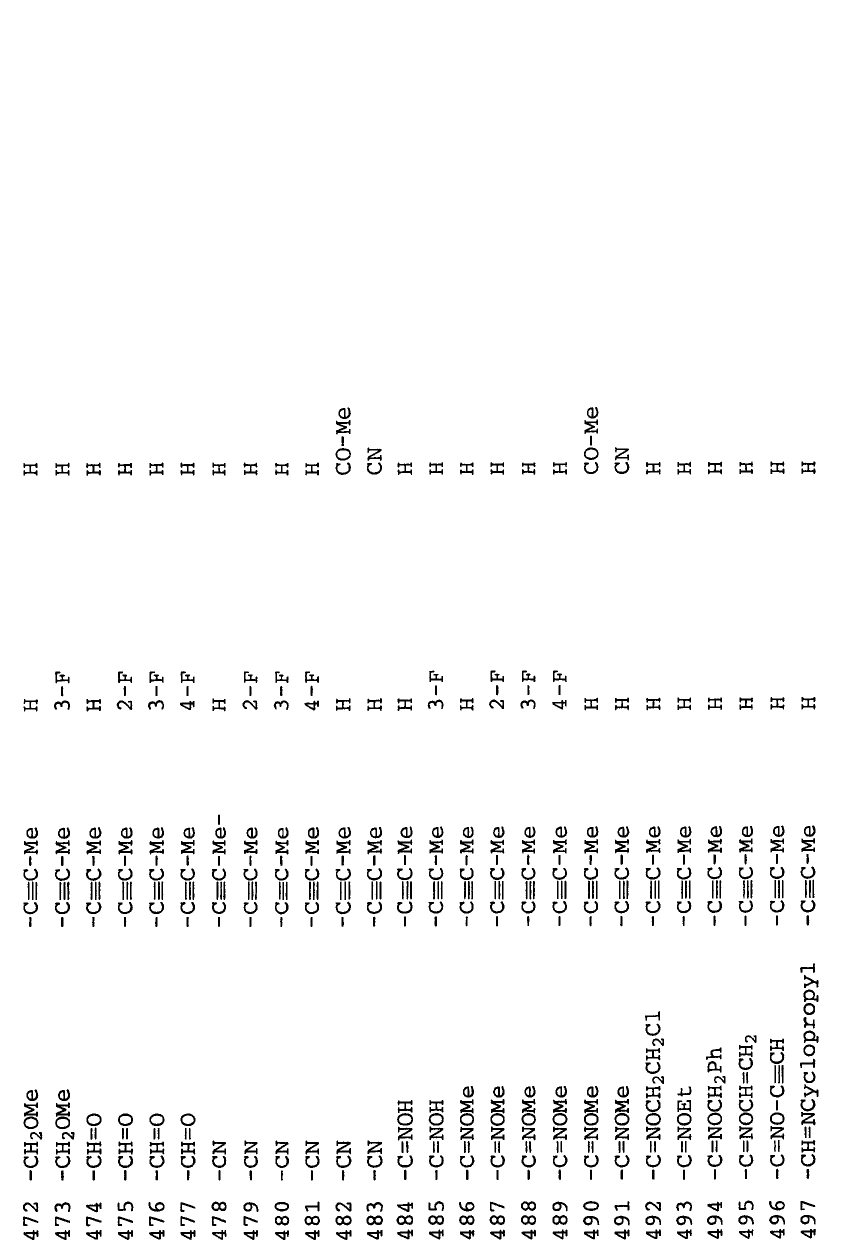

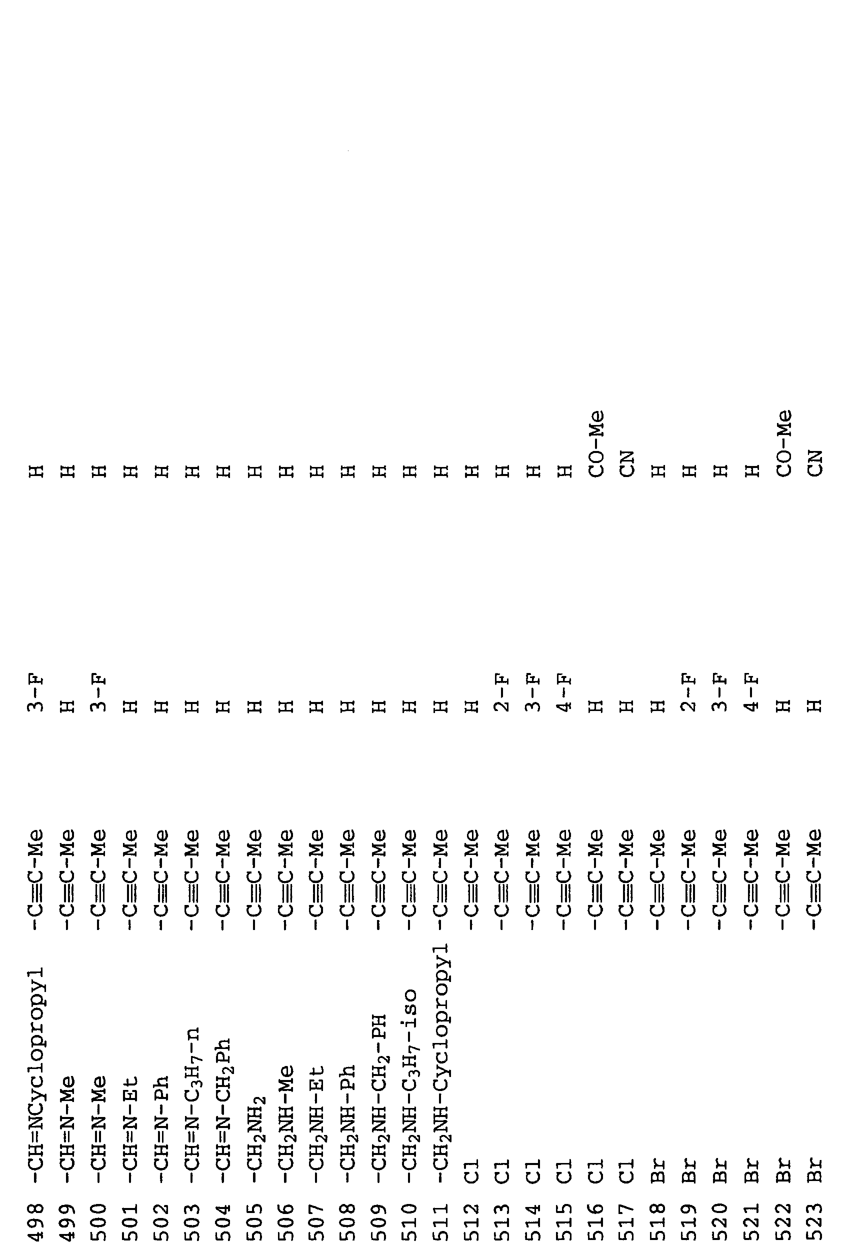

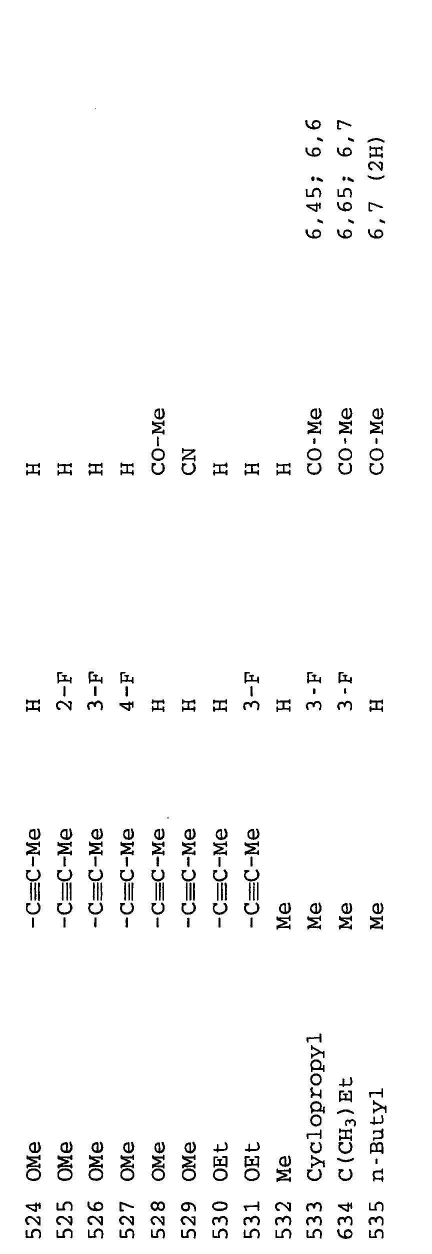

Die Verbindungen der Formel I eignen sich als Fungizide.

Die fungiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether- und Fettalkoholsulfaten, sowie Salze sulfatierter Hexa-, Hepta- und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Beispiele für solche Zubereitungen sind:
I. eine Lösung aus 90 Gew.-Teilen der Verbindung 7 und 10 Gew.-Teilen N-Methyl-a-pyrrolidon, die zur Anwendung in Form kleinster Tropfen geeignet ist;
II. eine Mischung aus 20 Gew.-Teilen der Verbindung 24, 80 Gew.Teilen Xylol, 10 Gew.-Teilen des Anlagerungsproduktes von 8 bis 10 Mol Ethylenoxid an 1 Mol Ölsäure-N-monoethanolamid, 5 Gew.Teilen Calciumsalz der Dodecylbenzolsulfonsäure, 5 Gew.- Teilen des Anlagerungsproduktes von 40 Mol Ethylenoxid an 1 Mol Ricinusöl; durch feines Verteilen der Lösung in Wasser erhält man eine Dispersion.
III. eine wäßrige Dispersion aus 20 Gew.-Teilen der Verbindung 25, 40 Gew.-Teilen Cyclohexanon, 30 Gew.-Teilen Isobutanol, 20 Gew.-Teilen des Anlagerungsproduktes von 40 mol Ethylenoxid an 1 mol Ricinusöl;
IV. eine wäßrige Dispersion aus 20 Gew.-Teilen der Verbindung 7, 25 Gew.-Teilen Cyclohexanol, 65 Gew.-Teilen einer Mineralölfraktion vom Siedepunkt 210 bis 280'C und 10 Gew.-Teilen des Anlagerungsproduktes von 40 mol Ethylenoxid an 1 mol Ricinusöl;
V. eine in einer Hammermühle vermahlene Mischung aus 80 Gew.-Teilen der Verbindung 24, 3 Gew.-Teilen des Natriumsalzes der Diisobutylnaphtalin-a-sulfonsäure, 10 Gew.-Teilen des Natriumsalzes einer Ligninsulfonsäure aus einer Sulfitablauge und 7 Gew.-Teilen pulverförmigem Kieselsäuregel; durch feines Verteilen der Mischung in Wasser erhält man eine Spritzbrühe;
VI. eine innige Mischung aus 3 Gew.-Teilen der Verbindung 28 und 97 Gew.-Teilen feinteiligem Kaolin; dieses Stäubemittel enthält 3 Gew.-% Wirkstoff;
VII. eine innige Mischung aus 30 Gew.-Teilen der Verbindung 7, 92 Gew.-Teilen pulverförmigem Kieselsäuregel und 8 Gew.-Teilen Paraffinöl, das auf die Oberfläche dieses Kieselsäuregels gesprüht wurde; diese Aufbereitung gibt dem Wirkstoff eine gute Haftfähigkeit;
VIII. eine stabile wäßrige Dispersion aus 40 Gew.-Teilen der Verbindung 24, 10 Gew.-Teilen des Natriumsalzes eines Phenolsulfonsäure-harnstoff-formaldehyd-Kondensates, 2 Gew.-Teilen Kieselgel und 48 Gew.-Teilen Wasser, die weiter verdünnt werden kann;
IX. eine stabile ölige Dispersion aus 20 Gew.-Teilen der Verbindung 28, 2 Gew.-Teilen des Calciumsalzes der Dodecylbenzolsulfonsäure, 8 Gew.-Teilen Fettalkohol-polyglykolether, 20 Gew.-Teilen des Natriumsalzes eines Phenolsulfonsäure-harnstoff-formaldehydKondensates und 68 Gew.-Teilen eines paraffinischen Mineralöls.

Die Verbindungen der Formel I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen I zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel,
   Dithiocarbamate und deren Derivate, wie
   Ferridimethyldithiocarbamat,
   Zinkdimethyldithiocarbamat,
   Zinkethylenbisdithiocarbamat,
   Manganethylenbisdithiocarbamat,
   Mangan-Zink-ethylendiamin-bis-dithiocarbamat,
   Tetramethylthiuramdisulfide,
   Ammoniak-Komplex von Zink-(N,N-ethylen-bis-dithiocarbamat),
   Ammoniak-Komplex von Zink-(N,N'-propylen-bis-dithiocarbamat),
   Zink-(N,N'-propylen-bis-dithiocarbamat),
   N,N-Polypropylen-bis-(thiocarbamoyl)-disulfid;
Nitroderivate, wie
   Dinitro-(1-methylheptyl)-phenylcrotonat,
   2-sec-Butyl-4,6-dinitrophenyl-3,3-dimethylacrylat,
   2-sec-Butyl-4,6-dinitrophenyl-isopropylcarbonat,
   5-Nitro-isophthalsäure-di-isopropylester;
heterocyclische Substanzen, wie
   2-Heptadecyl-2-imidazolin-acetat,
   2,4-Dichlor-6-(o-chloranilino)-s-triazin,
   O,O-Diethyl-phthalimidophosphonothioat,
   5-Amino-1-ßbis-(dimethylamino)-phosphinyl'-3-phenyl-1,2,4-triazol,
   2,3-Dicyano-1,4-dithioanthrachinon,
   2-Thio-1,3-dithioloß4,5-b'chinoxalin,
   1-(Butylcarbamoyl)-2-benzimidazol-carbaminsäuremethylester,
   2-Methoxycarbonylamino-benzimidazol,
   2-(Furyl-(2))-benzimidazol,
   2-(Thiazolyl-(4))-benzimidazol,
   N-(1,1,2,2-Tetrachlorethylthio)-tetrahydrophthalimid,
   N-Trichlormethylthio-tetrahydrophthalimid,
   N-Trichlormethylthio-phthalimid,
N-Dichlorfluormethylthio-N',N'-dimethyl-N-phenyl-schwefelsäurediamid,
   5-Ethoxy-3-trichlormethyl-1,2,3-thiadiazol,
   2-Rhodanmethylthiobenzthiazol,
   1,4-Dichlor-2,5-dimethoxybenzol,
   4-(2-Chlorphenylhydrazono)-3-methyl-5-isoxazolon,
   Pyridin-2-thio-1-oxid,
   8-Hydroxychinolin bzw. dessen Kupfersalz,
2,3-Dihydro-5-carboxanilido-6-methyl-1,4-oxathiin,
   2,3-Dihydro-5-carboxanilido-6-methyl-1,4-oxathiin-4,4-dioxid,
   2-Methyl-5,6-dihydro-4H-pyran-3-carbonsäure-anilid,
   2-Methyl-furan-3-carbonsäureanilid,
   2,5-Dimethyl-furan-3-carbonsäureanilid,
   2,4,5-Trimethyl-furan-3-carbonsäureanilid,
   2,5-Dimethyl-furan-3-carbonsäurecyclohexylamid,
   N-Cyclohexyl-N-methoxy-2,5-dimethyl-furan-3-carbonsäureamid,
   2-Methyl-benzoesäure-anilid,
   2-Iod-benzoesäure-anilid,
   N-Formyl-N-morpholin-2,2,2-trichlorethylacetal,
   Piperazin-1,4-diylbis-(1-(2,2,2-trichlor-ethyl)-formamid,
   1-(3,4-Dichloranilino)-1-formylamino-2,2,2-trichlorethan,
   2,6-Dimethyl-N-tridecyl-morpholin bzw. dessen Salze,
   2,6-Dimethyl-N-cyclododecyl-morpholin bzw. dessen Salze,
N-[3-(p-tert.-Butylphenyl)-2-methylpropyl]-cis-2,6-dimethylmorpholin,
   N-[3-(p-tert.-Butylphenyl)-2-methylpropyl]-piperidin,
   1-[2-(2,4-Dichlorphenyl)-4-ethyl-1,3-dioxolan-2-yl-ethyl]-1H-1,2,4-triazol
   1-[2-(2,4-Dichlorphenyl)-4-n-propyl-1,3-dioxolan-2-yl-ethyl]-1H-1,2,4-triazol
   N-(n-Propyl)-N-(2,4,6-trichlorphenoxyethyl)-N'-imidazol-yl-harnstoff,
   1-(4-Chlorphenoxy)-3,3-dimethyl-1-(1H-1,2,4-triazol-1-yl)-2-butanon,
   α-(2-Chlorphenyl)-α-(4-chlorphenyl)-5-pyrimidin-methanol,
   5-Butyl-2-dimethylamino-4-hydroxy-6-methyl-pyrimidin,
   Bis-(p-chlorphenyl)-3-pyridinmethanol,
   1,2-Bis-(3-ethoxycarbonyl-2-thioureido)-benzol,
   1,2-Bis-83-methoxycarbonyl-2-thioureido)-benzol,
sowie verschiedene Fungizide, wie
   Dodecylguanidinacetat,
   3-[3-(3,5-Dimethyl-2-oxycyclohexyl)-2-hydroxyethyl)]glutarimid,
   Hexachlorbenzol,
   DL-Methyl-N-(2,6-dimethyl-phenyl)-N-furoyl(2)-alaninat,
   DL-N-(2,6-Dimethyl-phenyl)-N-(2'-methoxyacetyl)-alanin-methylester,
   N-(2,6-Dimethylphenyl)-N-chloracetyl-D,L-2-aminobutyrolacton,
   DL-N-(2,6-Dimethylphenyl)-N-(phenylacetyl)-alaninmethylester,
   5-Methyl-5-vinyl-3-(3,5-dichlorphenyl)-2,4-dioxo-1,3-oxazolidin,
   3-[3,5-Dichlorphenyl(-5-methyl-5-methoxymethyl]-1,3-oxazolidin-2,4-dion,
   3-(3,5-Dichlorhenyl)-1-isopropylcarbamoylhydantoin,
N-(3,5-Dichlorphenyl)-1,2-dimethylcyclopropan-1,2-dicarbonsäureimid,
   2-Cyano-[N-(ethylaminocarbonyl)-2-methoximino]-acetamid,
   1-[2-(2,4-Dichlorphenyl)-pentyl]-1H-1,2,4-triazol,
   2,4-Difluor-α-(1H-1,2,4-triazolyl-1-methyl)-benzhydrylalkohol,
   N-(3-Chlor-2,6-dinitro-4-trifluormethyl-phenyl)-5-trifluormethyl-3-chlor-2-aminopyridin,
   1-(bis-(4-Fluorphenyl)-methylsilyl)-methyl)-1H-1,2,4-triazol.

### Anwendungsbeispiel

Als Vergleichswirkstoffe wurden 2-(2,4-Dimethylanilino)-3-cyano-4,6-dimethylpyridin (A) und 2-(4-Methoxyanilino)-3-cyano-4,6-dimethylpyridin (B) - beide bekannt aus DE 3 731 626 - verwendet.

### Anwendungsbeispiel 1

### Wirksamkeit gegen Botrytis cinerea an Paprikaschoten

Scheiben von grünen Paprikaschoten wurden mit wäßriger Wirkstoffaufbereitung, die 80 % Wirkstoff und 20 % Emulgiermittel in der Trockensubstanz enthielt, tropfnaß besprüht. 2 Stunden nach dem Antrocknen des Spritzbelages wurden die Fruchtscheiben mit einer Sporensuspension von Botrytis cinerea, die 1,7 x 10⁶ Sporen pro ml einer 2 %igen Biomalzlösung enthielt, inokuliert. Die inokulierten Fruchtscheiben wurden anschließend in feuchten Kammern bei 18°C für 4 Tage inkubiert. Dann erfolgte visuell die Auswertung der Botrytis-Entwicklung auf den befallenen Fruchtscheiben.

Das Ergebnis zeigt, daß der Wirkstoff 532 bei der Anwendung als 125 ppm Wirkstoff enthaltende Spritzbrühe eine bessere fungizide Wirkung zeigt (5 % Befall der Früchte) als die bekannten Vergleichswirkstoffe A und B (90 % Befall der Früchte).

## Patentansprüche

1. Verfahren zur Bekämpfung von Schädlingen, dadurch gekennzeichnet, daß man die Schädlinge oder die von Befall mit Schädlingen bedrohten Pflanzen oder ihre Umgebung oder ihre Saatgüter oder vor Schädlingsbefall zu schutzenden Materialien mit einer wirksamen Menge eines Wirkstoffs behandelt, der ein 2-Anilinopyridin der Formel I ist, 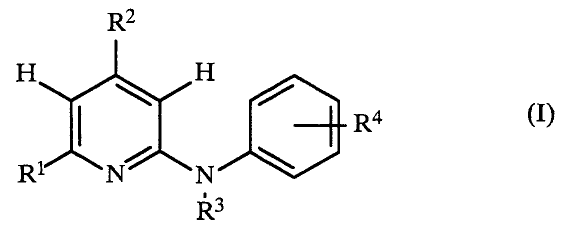 in der die Substituenten die folgenden Bedeutungen haben:
R¹ C₁-C₆-Alkyl, C₂-C₄-Alkenyl, C₂-C₄-Alkinyl, C₁-C₆-Halogenalkyl, C₁-C₆-Alkoxy-C₁-C₆-alkyl, C₁-C₆-Alkylthio-C₁-C₆-alkyl oder C₃-C₆-Cycloalkyl;
durch C₁-C₂-Alkyl oder Halogen bis zu dreifach gleich oder verschieden substituiertes C₃-C₆-Cycloalkyl, C₁-C₆-Alkoxy oder C₁-C₆-Halogenalkoxy;
durch Hydroxy substituiertes C₁-C₂-Alkyl, C₂-C₄-Alkenyloxy oder C₂-C₄-Alkinyloxy;
Halogen, CN, SCN, Formyl, CH=NOR⁵, CH=NR⁶, CH₂NHR⁶
R⁵ Wasserstoff, C₁-C₈-Alkyl;
C₁-C₄-Alkyl, das durch Halogen, Hydroxy, Cyano, C₁-C₄-Alkoxy, COOC₁-C₃-Alkyl, oder durch Phenyl substituiert ist, wobei das Phenyl durch Halogen, C₁-C₃-Alkyl, C₁-C₃-Alkoxy, oder Nitro substituiert sein kann;
C₃-C₆-Alkenyl, durch Halogen substituiertes C₃-C₆-Alkenyl, C₃-C₆-Alkinyl, durch Halogen substituiertes C₃-C₆-Alkinyl,
Phenyl oder 1 bis 3-fach durch Halogen, C₁-C₃-Alkyl, C₁-C₃-Alkoxy oder Nitro substituiertes Phenyl oder einen Acylrest COR⁷, worin
R⁷ C₁-C₆-Alkyl oder durch Halogen oder C₁-C₃-Alkoxy substituiertes C₁-C₆-Alkyl, C₃-C₆-Alkenyl, oder durch Halogen substituiertes C₃-C₆-Alkenyl bedeutet;
R⁶ Wasserstoff, C₁-C₆-Alkyl,
C₁-C₄-Alkyl, das durch Halogen, Hydroxy, Cyano, C₁-C₄-Alkoxy, C₁-C₄-Alkylamino, C₃-C₆-Cycloalkyl, oder durch Phenyl substituiert ist, wobei das Phenyl durch Halogen, C₁-C₃-Alkyl, C₁-C₃-Alkoxy, oder Nitro substituiert sein kann;
C₃-C₆-Cycloalkyl, oder durch Methyl substituiertes C₃-C₆-Cycloalkyl; C₃-C₆-Alkenyl oder durch Halogen substi-tuiertes C₃-C₆-Alkenyl; C₃-C₆-Alkinyl, oder durch Halogen substituiertes C₃-C₆-Alkinyl;
Phenyl oder durch Halogen, C₁-C₃-Alkyl, C₁-C₃-Alkoxy, C₁-C₂-Halogenalkyl, C₁-C₂-Halogenalkoxy, oder Nitro substituiertes Phenyl;
R² C₁-C₆-Alkyl C₂-C₄-Alkenyl C₂-C₄-Alkinyl C₁-C₆-Halogenalkyl, C₃-C₆-Cycloalkyl, durch C₁-C₂-Alkyl oder Halogen bis zu dreifach gleich oder verschieden substituiertes C₃-C₆-Cycloalkyl,
R³ Wasserstoff, CN, S(O)ₙR⁸, wobei n = 0, 1, 2 bedeutet, oder COR⁹
R⁸ C₁-C₃-Alkyl, Phenyl, gegebenenfalls 1- bis 3-fach durch Halogen, C₁-C₂-Alkyl, C₁-C₂-Halogenalkyl, Methoxy, Nitro substituiert,
R⁹ Wasserstoff, C₁-C₆-Alkyl, C₁-C₆-Halogenalkyl, C₃-C₆-Cycloalkyl, Phenyl oder Benzyl, oder 1 bis 3-fach mit Halogen, C₁-C₃-Alkyl, C₁-C₃-Halogenalkyl, C₁-C₃-Alkoxy, Nitro oder Cyano substituiertes Phenyl oder durch diese Reste substituiertes Benzyl,
R⁴ Wasserstoff, Halogen, C₁-C₃-Alkyl, C₁-C₂-Halogenalkyl, C₁-C₃-Alkoxy oder C₁-C₃-Halogenalkoxy, Cyano.

2. Anilinopyridine der Formel I gemäß Anspruch 1, ausgenommen diejenigen Verbindungen, in denen
a) gleichzeitig
R¹ = C₁-C₄-Alkyl, C₁-C₄-Alkoxy, C₁-C₄-Halogenalkyl, C₁-C₄-Halogenalkoxy, C₃-C₆-Cycloalkyl, Halogen oder Cyano,
R² = C₁-C₄-Alkyl, C₁-C₄-Halogenalkyl oder C₃-C₆-Cycloalkyl und
R³ = Wasserstoff,
und
b)
R¹ = Methyl,
R² = Methyl,
R³ = Cyano und
R⁴ = Wasserstoff.

3. Verbindung der Formel I gemäß Anspruch 1, in der R¹ Cyclopropyl, R² Methyl, R⁴ Wasserstoff und R³ Tosylsulfonyl (1-CH₃-C₆H₄-4-SO₃) bedeuten.

4. Mittel zur Bekämpfung von Pilzen, enthaltend einen inerten Trägerstoff und eine fungizid wirksame Menge eines Anilinopyridins I gemäß Anspruch 2.

5. Verfahren zur Bekämpfung von Pilzen gemäß Anspruch 1.

6. Verfahren zur Bekämpfung von phytopathogenen Pilzen gemäß Anspruch 1.

7. Verfahren zur Bekämpfung von Botrytis cinerea gemäß Anspruch 1.

8. Verwendung der Verbindungen der Formel I gemäß Anspruch 1 zur Herstellung von Mitteln zur Bekämpfung von Schädlingen.

## Claims

1. A method of controlling pests, which comprises treating the pests or the plants threatened by attack with pests or their environment or their seeds or materials to be protected from pest attack with an effective amount of an active compound which is a 2-anilinopyridine of the formula I 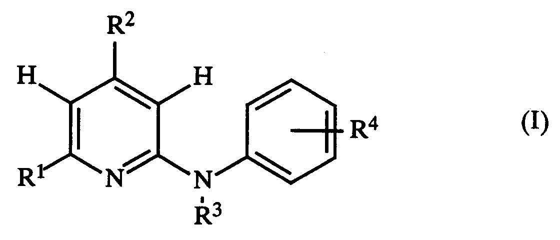 where the substituents have the following meanings:
R¹ is C₁-C₆-alkyl, C₂-C₄-alkenyl, C₂-C₄-alkynyl, C₁-C₆-haloalkyl, C₁-C₆-alkoxy-C₁-C₆-alkyl, C₁-C₆-alkylthio-C₁-C₆-alkyl or C₃-C₆-cycloalkyl;
C₃-C₆-cycloalkyl, C₁-C₆-alkoxy or C₁-C₆-haloalkoxy substituted up to three times by identical or different C₁-C₂-alkyl or halogen substituents;
C₁-C₂-alkyl, C₂-C₄-alkenyloxy or C₂-C₄-alkynyloxy substituted by hydroxyl;
halogen, CN, SCN, formyl, CH=NOR⁵, CH=NR⁶, CH₂NHR⁶
R⁵ is hydrogen, C₁-C₈-alkyl;
C₁-C₄-alkyl which is substituted by halogen, hydroxyl, cyano, C₁-C₄-alkoxy, COOC₁-C₃-alkyl or by phenyl, it being possible for the phenyl to be substituted by halogen, C₁-C₃-alkyl, C₁-C₃-alkoxy or nitro;
C₃-C₆-alkenyl, C₃-C₆-alkenyl substituted by halogen, C₃-C₆-alkynyl, C₃-C₆-alkynyl substituted by halogen,
phenyl or phenyl substituted one to three times by halogen, C₁-C₃-alkyl, C₁-C₃-alkoxy or nitro or
an acyl radical COR⁷ where
R⁷ is C₁-C₆-alkyl or C₁-C₆-alkyl substituted by halogen or C₁-C₃-alkoxy, C₃-C₆-alkenyl or C₃-C₆-alkenyl substituted by halogen;
R⁶ is hydrogen, C₁-C₆-alkyl,
C₁-C₄-alkyl which is substituted by halogen, hydroxyl, cyano, C₁-C₄-alkoxy, C₁-C₄-alkylamino, C₃-C₆-cycloalkyl or by phenyl, it being possible for the phenyl to be substituted by halogen, C₁-C₃-alkyl, C₁-C₃-alkoxy or nitro;
C₃-C₆-cycloalkyl or C₃-C₆-cycloalkyl substituted by methyl; C₃-C₆-alkenyl or C₃-C₆-alkenyl substituted by halogen; C₃-C₆-alkynyl or C₃-C₆-alkynyl substituted by halogen;
phenyl or phenyl substituted by halogen, C₁-C₃-alkyl, C₁-C₃-alkoxy, C₁-C₂-haloalkyl, C₁-C₂-haloalkoxy or nitro;
R² is C₁-C₆-alkyl, C₂-C₄-alkenyl, C₂-C₄-alkynyl, C₁-C₆-haloalkyl, C₃-C₆-cycloalkyl or C₃-C₆-cycloalkyl substituted up to three times by identical or different C₁-C₂-alkyl or halogen substituents,
R³ is hydrogen, CN, S(O)ₙR⁸ where n = 0, 1 or 2, or COR⁹
R⁸ is C₁-C₃-alkyl, or phenyl, optionally substituted 1 to 3 times by halogen, C₁-C₂-alkyl, C₁-C₂-haloalkyl, methoxy or nitro,
R⁹ is hydrogen, C₁-C₆-alkyl, C₁-C₆-haloalkyl, C₃-C₆-cycloalkyl, phenyl or benzyl, or phenyl substituted 1 to 3 times by halogen, C₁-C₃-alkyl, C₁-C₃-haloalkyl, C₁-C₃-alkoxy, nitro or cyano, or benzyl substituted by these radicals,
R⁴ is hydrogen, halogen, C₁-C₃-alkyl, C₁-C₂-haloalkyl, C₁-C₃-alkoxy or C₁-C₃-haloalkoxy, or cyano.

2. An anilinopyridine of the formula I as in claim 1, with the exception of those compounds in which
a) at one and the same time
R¹ = C₁-C₄-alkyl, C₁-C₄-alkoxy, C₁-C₄-haloalkyl, C₁-C₄-haloalkoxy, C₃-C₆-cycloalkyl, halogen or cyano,
R² = C₁-C₄-alkyl, C₁-C₄-haloalkyl or C₃-C₆-cycloalkyl and
R³ = hydrogen,
and
b)
R¹ = methyl,
R² = methyl,
R³ = cyano and
R⁴ = hydrogen.

3. A compound of the formula I as in claim 1, where R¹ is cyclopropyl, R² is methyl, R⁴ is hydrogen and R³ is tosylsulfonyl (1-CH₃-C₆H₄-4-SO₃)

4. A composition for controlling fungi, containing an inert carrier and a fungicidally effective amount of an anilinopyridine I as claimed in claim 2.

5. A method for controlling fungi as claimed in claim 1.

6. A method for controlling phytopathogenic fungi as claimed in claim 1.

7. A method for controlling Botrytis cinerea as claimed in claim 1.

8. The use of the compounds of the formula I as in claim 1, for the production of compositions for controlling pests.

## Revendications

1. Procédé pour combattre les parasites, caractérisé par le fait que l'on traite les parasites ou les végétaux menacés d'une attaque par les parasites ou leur entourage ou leurs semences ou bien les matières à protéger contre une attaque par les parasites par une quantité efficace d'une substance active consistant en une 2-anilinopyridine de formule I 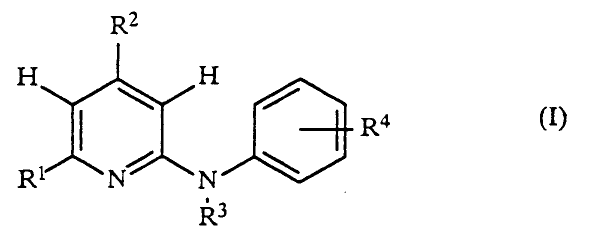 dans laquelle les symboles ont les significations suivantes :
R¹ représente un groupe alkyle en C1-C6, alcényle en C2-C4, alcynyle en C2-C4, halogénoalkyle en C1-C6, (alcoxy en C1-C6)alkyle en C1-C6, (alkyle en C1-C6)thioalkyle en C1-C6 ou cycloalkyle en C3-C6 ;
cycloalkyle en C3-C6, alcoxy en C1-C6 ou halogénoalcoxy en C1-C6 portant jusqu'à trois substituants identiques ou différents choisis parmi les groupes alkyle en C1-C2 et les halogènes ;
un groupe alkyle en C1-C2, alcényloxy en C2-C4 ou alcynyloxy en C2-C4 substitué par un groupe hydroxy ;
un halogène, un groupe CN, SCN, formyle, CH=NOR⁵, CH=NR⁶, CH₂NHR⁶,
R⁵ représente l'hydrogène, un groupe alkyle en C1-C8 ;
un groupe alkyle en C1-C4 substitué par des halogènes, des groupes hydroxy, cyano, alcoxy en C1-C4, COO-alkyle en C1-C3 ou phényle, ce dernier pouvant lui-même être substitué par des halogènes, des groupes alkyle en C1-C3, alcoxy en C1-C3 ou nitro ;
un groupe alcényle en C3-C6, alcényle en C3-C6 substitué par des halogènes,
alcynyle en C3-C6, alcynyle en C3-C6 substitué par des halogènes,
un groupe phényle ou un groupe phényle portant un à trois substituants choisis parmi les halogènes, les groupes alkyle en C1-C3, alcoxy en C1-C3 et nitro, ou bien
un groupe acyle OR⁷ dans lequel
R⁷ représente un groupe alkyle en C1-C6 ou un groupe alkyle en C1-C6 substitué par des halogènes ou des groupes alcoxy en C1-C3, un groupe alcényle en C3-C6 ou un groupe alcényle en C3-C6 substitué par des halogènes ;
R⁶ représente l'hydrogène, un groupe alkyle en C1-C6,
un groupe alkyle en C1-C4 substitué par des halogènes, des groupes hydroxy, cyano, alcoxy en C1-C4, alkylamino en C1-C4, cycloalkyle en C3-C6 ou phényle, ce dernier pouvant lui-même être substitué par des halogènes, des groupes alkyle en C1-C3, alcoxy en C1-C3 ou nitro ;
un groupe cycloalkyle en C3-C6 ou un groupe cycloalkyle en C3-C6 substitué par un groupe méthyle ; un groupe alcényle en C3-C6 ou alcényle en C3-C6 substitué par des halogènes ; un groupe alcynyle en C3-C6 ou un groupe alcynyle en C3-C6 substitué par des halogènes ;
un groupe phényle ou un groupe phényle substitué par des halogènes, des groupes alkyle en C1-C3, alcoxy en C1-C3, halogénoalkyle en C1-C2, halogénoalcoxy en C1-C2 ou nitro ;
R² représente un groupe alkyle en C1-C6, alcényle en C2-C4, alcynyle en C2-C4, halogénoalkyle en C1-C6, cycloalkyle en C3-C6, cycloalkyle en C3-C6 portant jusqu'à trois substituants identiques ou différents choisis parmi les groupes alkyle en C1-C2 et les halogènes,
R³ représente l'hydrogène, un groupe CN, S(O)ₙR⁸ dans lequel n = 0, 1, 2, ou un groupe COR⁹,
R⁸ représente un groupe alkyle en C1-C3, phényle, lui-même éventuellement mono- à tri-substitué par des halogènes, des groupes alkyle en C1-C2, halogénoalkyle en C1-C2, méthoxy, nitro,
R⁹ représente l'hydrogène, un groupe alkyle en C1-C6, halogénoalkyle en C1-C6, cycloalkyle en C3-C6, phényle ou benzyle ou bien phényle ou benzyle portant un à trois substituants choisis parmi les halogènes, les groupes alkyle en C1-C3, halogénoalkyle en C1-C3, alcoxy en C1-C3, nitro ou cyano,
R⁴ représente l'hydrogène, un halogène, un groupe alkyle en C1-C3, halogénoalkyle en C1-C2, alcoxy en C1-C3 ou halogénoalcoxy en C1-C3, cyano.

2. Anilinopyridines de formule I selon la revendication 1, à l'exception des composés pour lesquels
a) simultanément
R¹ = alkyle en C1-C4, alcoxy en C1-C4, halogénoalkyle en C1-C4, halogénoalcoxy en C1-C4, cycloalkyle en C3-C6, halogène ou cyano,
R² = alkyle en C1-C4, halogénoalkyle en C1-C4 ou cycloalkyle en C3-C6 et
R³ = hydrogène,
et
b) R¹ = méthyle,
R² = méthyle,
R³ = cyano et
R⁴ = hydrogène.

3. Composé de formule I selon la revendication 1, dans laquelle R¹ représente un groupe cyclopropyle, R² un groupe méthyle, R⁴ l'hydrogène et R³ un groupe toluènesulfonyle (1-CH₃-C₆H₄-4-SO₃).

4. Produit pour combattre les mycètes, contenant un véhicule inerte et une quantité fongicide efficace d'une anilinopyridine I selon la revendication 2.

5. Procédé pour combattre les mycètes selon la revendication 1.

6. Procédé pour combattre les mycètes phytopathogènes selon la revendication 1.

7. Procédé pour combattre Botrytis cinerea selon la revendication 1.

8. Utilisation des composés de formule I selon la revendication 1 pour la préparation de produits servant à combattre les parasites.
